(19) Europäisches Patentamt
European Patent Office
Office européen des brevets

(11) Veröffentlichungsnummer: **0 116 708**
**B1**

(12) # EUROPÄISCHE PATENTSCHRIFT

(45) Veröffentlichungstag der Patentschrift: **14.05.86**

(21) Anmeldenummer: **83112562.0**

(22) Anmeldetag: **14.12.83**

(51) Int. Cl.⁴: **C 07 D 413/04,** C 07 D 413/14, C 07 D 417/04, C 07 D 417/14, A 61 K 31/44 // (C07D413/04, 211:90, 271:06),(C07D413/04, 211:90, 271:10),(C07D417/04, 211:90, 277:56),(C07D417/04, 211:90, 285:08)

(54) **Neue substituierte 1,4-Dihydropyridine, Verfahren zu ihrer Herstellung und ihre Verwendung als Arzneimittel.**

(30) Priorität: **20.12.82 DE 3247118**

(43) Veröffentlichungstag der Anmeldung: **29.08.84 Patentblatt 84/35**

(45) Bekanntmachung des Hinweises auf die Patenterteilung: **14.05.86 Patentblatt 86/20**

(84) Benannte Vertragsstaaten: **AT BE CH DE FR GB IT LI NL SE**

(56) Entgegenhaltungen:
**EP - A - 0 042 089**
**DE - A - 1 963 186**
**DE - A - 2 005 116**
**DE - A - 2 747 513**
**DE - A - 2 847 236**
**GB - A - 1 271 302**
**US - A - 4 144 343**
**US - A - 4 260 765**

**CHEMICAL ABSTRACTS, Vol. 93, No. 23, 8. Dezember 1980, Columbus, Ohio, USA BAYER A.-G. "1,4-Dihydropyridine-carboxylic acid derivatives" Seite 516, Spalte 1, Abstract-No. 220576j**
**CHEMICAL ABSTRACTS, Vol. 94, No. 11, 16. März 1981, Columbus, Ohio, USA INSTITUTE OF ORGANIC SYNTHESIS, ACADEMY OF SCIENCES**

(73) Patentinhaber: **CASSELLA Aktiengesellschaft, Hanauer Landstrasse 526, D-6000 Frankfurt am Main 61 (DE)**

(72) Erfinder: **Schönafinger, Karl, Dr., Holunderweg 8, D-8755 Alzenau (DE)**
Erfinder: **Bohn, Helmut, Dr., Kranzbergring 11, D-6369 Schöneck (DE)**
Erfinder: **Just, Melitta, Dr., Hüttenberg 6, D-6369 Schöneck 1 (DE)**
Erfinder: **Martorana, Piero, Dr., Kaiser-Friedrich-Promenade 108, D-6380 Bad Homburg (DE)**

(74) Vertreter: **Urbach, Hans-Georg, Dr. et al, Hanauer Landstrasse 526, D-6000 Frankfurt am Main 61 (DE)**

(56) Entgegenhaltungen: (Fortsetzung)
**"1,4-Dihydropyridine-3-carbothioic acid derivatives" Seite 711, Spalte 2, Abstract-No. 83961d**

**EP 0 116 708 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die Erfindung betrifft neue substituierte 1,4-Dihydropyridine der Formel I

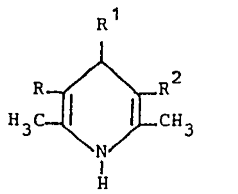

(I)

worin

R $-CO_2R^3$, Cyano oder eine der unter $R^2$ angegebenen Bedeutungen,

$R^1$ Pyridyl oder Thienyl, wobei der Pyridyl- oder Thienyl-Rest gegebenenfalls 1 oder 2 gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyano besitzt; Phenyl, das gegebenenfalls 1 oder 2 gleiche oder verschiedene Substituenten aus der Gruppe Halogen, Nitro, Cyano, Trifluormethyl, Alkyl mit 1 bis 4 C-Atomen, Alkoxy mit 1 bis 4 C-Atomen besitzt,

$R^2$ den Rest eines 5-gliedrigen Rings mit mindestens einer Doppelbindung und mindestens 2 Heteroatomen oder Heteroatomgruppierungen aus der Reihe O, N, NH, S, wobei der 5-gliedrige Ring gegebenenfalls 1 oder 2 gleiche oder verschiene Substituenten aus der Gruppe Alkyl mit 1 bis 4 C-Atomen, Alkylthio mit 1 bis 4 C-Atomen, Aralkyl mit insgesamt 7 bis 9 C-Atomen, Alkoxyalkyl mit insgesamt 2 bis 5 C-Atomen, Cycloalkyl mit 5 oder 6 C-Atomen, Aminocarbonylmethylthio, Methoxy-carbonyl, Ethoxy-carbonyl, Phenyl besitzt,

$R^3$ Alkyl mit 1 bis 6 C-Atomen, Alkoxyalkyl mit 3 bis 8 C-Atomen, Dialkylaminoalkyl mit insgesamt 4 bis 9 C-Atomen, N-Aralkyl-N-alkylaminoalkyl mit insgesamt 10 bis 14 C-Atomen, Cycloalkyl mit 5 oder 6 C-Atomen bedeuten, sowie ihre Säureadditionssalze.

Die Erfindung betrifft auch ein Verfahren zur Herstellung der Verbindungen der Formel I sowie ihre Verwendung als Arzneimittel.

Die genannten Reste Alkyl, Alkoxy sind, auch wenn sie in Kombinationen miteinander oder in anderen Resten, wie z. B. Alkoxyalkyl, Aralkyl, Dialkylaminoalkyl, Alkoxycarbonyl oder als Substituenten für andere Reste vorkommen, geradkettig oder verzweigt. Sofern für sie oder für die sie enthaltenden Gruppierungen nicht bereits vorstehend Bereiche für ihre Kohlenstoffzahl angegeben sind, enthalten sie normalerweise 1 bis 4 C-Atome.

Die genannten Aralkylreste sind insbesondere Phenalkylreste, nämlich Phenylpropyl, Phenylethyl oder Benzyl, von denen Phenylethyl und insbesondere Benzyl bevorzugt sind.

Halogen bedeutet in der Regel Chlor, Brom oder Fluor, vorzugsweise Chlor oder Brom, ganz besonders bevorzugt Chlor.

R bedeutet vor allem eine der unter $R^2$ angegebenen Bedeutungen, wie z. B. Oxadiazolyl, insbesondere 1,3,4-Oxadiazol-2-yl oder 3-Benzyl-1,2,4-Oxadiazol-5-yl. Vorzugsweise bedeutet R = $-CO_2R^3$.

$R^1$ kann ein 2-, 3- oder 4-Pyridylrest oder ein 2- oder 3-Thienylrest sein, wobei diese Reste ein oder zwei gleiche oder verschiedene Substituenten besitzen können.

$R^1$ bedeutet vorzugsweise Phenyl, das gegebenenfalls ein oder zwei gleiche oder verschiedene Substituenten, vorzugsweise aus der Reihe Chlor, Brom, Fluor, Nitro, Cyano, Methyl, Methoxy, Trifluormethyl, besitzt. Beispiel für derartige für $R^1$ stehende Reste sind: Phenyl, 2-Chlorphenyl, 3-Chlorphenyl, 4-Chlorphenyl, 2,3-Dichlorphenyl, 2-Nitrophenyl, 3-Nitrophenyl, 3-Cyanophenyl, 3-Methoxyphenyl, 3-Trifluormethylphenyl, 2-Trifluormethylphenyl, o-Tolyl, m-Tolyl oder p-Tolyl.

Besonders bevorzugt bedeutet $R^1$ ein durch Cyan, Nitro oder Chlor monosubstituiertes oder durch Chlor disubstituiertes Phenyl, wobei die Substituenten vorzugsweise in 2- und/oder 3-Stellung des Phenylkerns vorhanden sind. Ganz besonders bevorzugt bedeutet $R^1$ 2-Nitrophenyl, 3-Nitrophenyl, 3-Cyanophenyl, 2-Chlorophenyl und 2,3-Dichlorophenyl.

$R^2$ kann z. B. ein Oxazolyl-, Thiazolyl-, Imidazolyl-, Triazolyl-, Oxadiazolyl-, Thiadiazolyl-Rest sein. Als Substituenten für die Reste $R^2$ kommen z. B. in Betracht: Methyl, Ethyl, n-Propyl, i-Propyl, n-Butyl, i-Butyl, tert.-Butyl, Benzyl, Methylthio, i-Propylthio, Methoxymethyl, 2-Methoxyethyl, Aminocarbonylmethylthio, Methoxy-carbonyl, Ethoxy-carbonyl, Cyclopentyl, Cyclohexyl, Phenyl. Bei den für $R^2$ stehenden Resten von 5-gliedrigen Ringen sind solche bevorzugt, die zwei Stickstoffatome und ein Sauerstoffatom und zwei Doppelbindungen enthalten, wie z. B. 1,3,4-Oxadiazol-2-yl, 1,2,4-Oxadiazol-5-yl, 1,2,4-Oxadiazol-3-yl. Bevorzugte Substituenten sind: Methyl, Ethyl, i-Propyl, tert.-Butyl, Benzyl, Methylthio, i-Propylthio, Aminocarbonylmethylthio, Methoxymethyl. Besonders bevorzugte Substituenten sind: Methyl, Ethyl und Benzyl. Besonders bevorzugt für $R^2$ sind 1,3,4-Oxadiazol-2-yl, 5-Methyl-1,3,4-oxadiazol-2-yl, 5-Ethyl-1,3,4-oxadiazol-2-yl, 3-Methyl-1,2,4-oxadiazol-5-yl, 3-Ethyl-1,2,4-oxadiazol-5-yl, 3-Benzyl-1,2,4-oxadiazol-5-yl.

Bei dem für $R^3$ stehenden N-Aralkyl-N-alkylaminoalkyl-Rest sitzt die N-Aralkyl-N-alkyl-aminogruppe, insbesondere an dem endständigen C-Atom des Alkylrestes, wie z. B.: 2-(N-Benzyl-N-methyl-amino)-ethyl, 2-(N-Phenylethyl-N-methylamino)-ethyl, 2-(N-Benzyl-N-ethyl-amino)-ethyl.

Vorzugsweise bedeutet $R^3$ Alkyl mit 1 bis 5 C-Atomen, Alkoxyalkyl mit 1 bis 4 C-Atomen im Alkoxyteil und 2 bis 4 C-Atomen im Alkylteil, Dialkylaminoalkyl mit insgesamt 3 bis 6 C-Atomen, wobei jede der die Aminogruppe substituierenden Alkylgruppen 1 bis 3 C-Atomen besitzen kann und wobei bei dem Alkoxyalkylrest die Alkoxygruppe und bei dem Dialkylaminoalkylrest die Dialkylaminogruppe, insbesondere an dem endständigen C-Atom des Alkylrestes, sitzen. Beispiele für derartige bevorzugte Reste $R^3$ sind: Methyl, Ethyl, n-Propyl, i-Propyl, n-Butyl, i-Butyl, sec.-Butyl, tert.-

Butyl, Neopentyl, 2-Methoxy-ethyl, 2-i-Propoxyethyl, 2-n-Butoxyethyl, 3-Methoxy-n-propyl, 2-Dimethylamino-ethyl. Ganz besonders bevorzugt bedeutet $R^3$ Methyl, n-Propyl, i-Propyl, n-Butyl, i-Butyl, tert.-Butyl, 2-Methoxy-ethyl und 2-i-Propoxyethyl.

Bevorzugte Verbindungen der Formel I sind solche, bei denen die Reste eine oder insbesondere mehrere der angegebenen bevorzugten Bedeutungen besitzen. Ganz besonders bevorzugte Verbindungen sind solche, bei denen die Reste eine oder insbesondere mehrere, vorzugsweise alle der angegebenen, besonders bevorzugten Bedeutungen besitzen.

Besonders bevorzugte Verbindungen der Formel I sind z. B. die Verbindungen der nachfolgenden Beispiel 1z, 2b, 2r, 2s, 2v und insbesondere 2n und 2z2.

Die substituierten 1,4-Dihydropyridine der Formel I können in Analogie zu der Herstellung anderer 1,4-Dihydropyridin-Verbindungen, ausgehend von Verbindungen der Formeln II bis IX

$H_3C-CO-CH_2-R^2$, $H_3N$, $R^1-CHO$,
(II) (III) (IV)

$H_3C-CO-CH_2-R$,
(V)

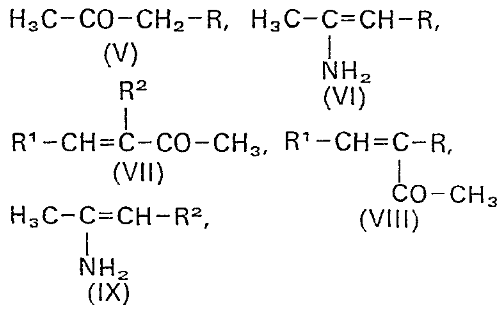
(VI) (VII) (VIII) (IX)

dadurch hergestellt werden, dass

a) 2 mol einer Verbindung der Formel II und 1 mol einer Verbindung der Formel IV und 1 mol einer Verbindung der Formel III oder
b) 1 mol einer Verbindung der Formel II und 1 mol einer Verbindung der Formel III und 1 mol einer Verbindung der Formel IV und 1 mol einer Verbindung der Formel V oder
c) 1 mol einer Verbindung der Formel II und 1 mol einer Verbindung der Formel IV und 1 mol einer Verbindung der Formel VI oder
d) 1 mol einer Verbindung der Formel VI und 1 mol einer Verbindung der Formel VII oder
e) 1 mol einer Verbindung der Formel VIII und 1 mol einer Verbindung der Formel II und 1 mol einer Verbindung der Formel III oder
f) 1 mol einer Verbindung der Formel V und 1 mol einer Verbindung der Formel III und 1 mol einer Verbindung der Formel VII oder
g) 1 mol einer Verbindung der Formel VIII und 1 mol einer Verbindung der Formel IX oder
h) 2 mol einer Verbindung der Formel IX und 1 mol einer Verbindung der Formel IV oder
i) 1 mol einer Verbindung der Formel V und 1 mol einer Verbindung der Formel IV und 1 mol einer Verbindung der Formel IX oder
k) 1 mol einer Verbindung der Formel VII und 1 mol einer Verbindung der Formel IX

miteinander umgesetzt werden und die erhaltene Verbindung gewünschtenfalls in an sich bekannter Weise in ein Säureadditionssalz überführt wird.

Ausgehend von den Verbindungen der Formeln II bis IX bestehen aber noch weitere mögliche Syntheseverfahren für die Verbindungen der Formel I. Die Verfahrensvarianten stellen Varianten oder Teilschritte der bekannten Hantzsch'schen Pyridinsynthese dar.

Die Umsetzung wird bei allen Varianten a) bis k) bei Raumtemperatur (20° C) oder insbesondere erhöhter Temperatur, z. B. in einem Bereich von 20 bis 120° C, durchgeführt. Vorzugsweise wird die Umsetzung bei allen Varianten a) bis k) bei der Rückflusstemperatur des verwendeten Lösungsmittels oder Lösungsmittelgemischs durchgeführt. Normalerweise erfolgt die Umsetzung bei Normaldruck, kann aber auch bei einem vom Normaldruck abweichenden Druck durchgeführt werden.

Die Umsetzungen werden in Wasser oder einem inerten organischen Lösungsmittel durchgeführt. Geeignete Lösungsmittel sind z. B. Alkohole, insbesondere solche mit 1 bis 6 C-Atomen, wie z. B. Methanol, Ethanol, i- und n-Propanol, i-, sec.- und tert.-Butanol, n-, i-, sec.-, tert.-Pentanol, m-Hexanol, Cyclopentanol, Cyclohexanol; Ether, insbesondere solche mit 2 bis 8 C-Atomen im Molekül, wie z. B. Diethylether, Methyl-ethyl-ether, Di-n-propyl-ether, Di-iso-propyl-ether, Methyl-n-butyl-ether, Ethylpropyl-ether, Di-butyl-ether, Tetrahydrofuran; 1,4-Dioxan, 1,2-Dimethoxyethan, Bis-β-methoxyethyl-ether; Polyether, wie z. B. Polyethylenglykole mit einem Molekulargewicht bis ca. 600; Oligoethylen-glykol-dimethylether, wie z. B. Pentaglyme; Glykole und teilweise veretherte Glykole, wie z. B. Ethylenglykol, Propylenglykol, Trimethylenglykol, Ethylenglykolmonomethyl-ether, Ethylenglykol-monoethyl-ether, Diethylenglykol-monoethyl-ether; Ketone, insbesondere solche mit 3 bis 10 C-Atomen im Molekül, wie z. B. Aceton, Methylethylketon, Methyl-n-propylketon, Diethylketon, 2-Hexanon, 3-Hexanon, Di-n-propylketon, Di-iso-propylketon, Di-iso-butylketon, Cyclopentanon, Cyclohexanon, Benzophenon, Acetophenon; aliphatische Kohlenwasserstoffe, wie z. B. niedrig- und hochsiedende Petrolether; aromatische Kohlenwasserstoffe, wie z. B. Benzol, Toluol, o-, m- und p-Xylol, Pyridin; halogenierte aliphatische oder aromatische Kohlenwasserstoffe, wie z. B. Methylenchlorid, Chloroform, Tetrachlorkohlenstoff, Ethylenchlorid, Chlorbenzol, Dichlorbenzol; Nitrile, wie z. B. Acetonitril; Amide, wie z. B. Dimethylformamid, N-Methyl-pyrrolidon; Hexamethylphosphorsäuretriamid; Sulfoxide, wie z. B. Dimethylsulfoxid; Wasser. Auch Gemische verschiedener Lösungsmittel können verwendet werden. Alkohole oder Gemische von Alkoholen mit Wasser sind in der Regel bevorzugt.

Von den oben genannten Verfahrensvarianten für die Herstellung der Verbindungen der Formel I ist die Verfahrensvariante c) bevorzugt.

Die zur Herstellung der Verbindungen der Formel I benötigten Ausgangsprodukte der Formeln II

bis IX sind bekannt oder können leicht nach den für die jeweilige Verbindungsklasse bekannten Verfahren hergestellt werden. Die Enamino-Verbindungen der Formeln VI und IX können, falls sie nicht bereits bekannt sind, z. B. hergestellt werden nach A.C. Cope, J. Amer. Chem. Soc., *67,* 1017 (1945).

Als Beispiele für Enamino-Verbindungen der Formeln VI und IX seien genannt: 3-Aminocrotonsäuremethylester, 3-Aminocrotonsäureethylester, 3-Aminocrotonsäure-i-propylester, 3-Aminocrotonsäure-n-propylester, 3-Aminocrotonsäure-(2-methoxy-ethyl)-ester, 3-Aminocrotonsäure-(3-methoxy-propyl)-ester, 3-Aminocrotonsäure-(2-butoxy-ethyl)-ester, 3-Aminocrotonsäure-cyclopentylester, 3-Aminocrotonsäure-cyclohexylester, 2-(2-Aminopropen-1-yl)-4-methyl-5-ethoxycarbonyl-thiazol, 2-(2-Aminopropen-1-yl)-thiazol, 2-(2-Aminopropen-1-yl)-4-phenyl-thiazol, 5-(2-Aminopropen-1-yl)-3-methyl-1,2,4-oxadiazol, 5-(2-Aminopropen-1-yl)-3-ethyl-1,2,4-oxadiazol, 5-(2-Aminopropen-1-yl)-3-tert.-butyl-1,2,4-oxadiazol, 5-(2-Aminopropen-1-yl)-3-benzyl-1,2,4-oxadiazol, 2-(2-Aminopropen-1-yl)-1,3,4-oxadiazol, 2-(2-Aminopropen-1-yl)-5-aminocarbonylmethylthio-1,3,4-oxadiazol, 2-(2-Aminopropen-1-yl)-5-methyl-1,3,4-oxadiazol, 3-(2-Aminopropen-1-yl)-1,2,4-oxadiazol, 3-(2-Aminopropen-1-yl)-5-methyl-1,2,4-oxadiazol, 3-(2-Aminopropen-1-yl)-5-benzyl-1,2,4-oxadiazol, 5-(2-Aminopropen-1-yl)-1,2,4-thiadiazol, 5-(2-Aminopropen-1-yl)-3-methylthio-1,2,4-thiadiazol.

Die als Ausgangskomponenten dienenden Aldehyde der Formel IV können, falls sie nicht bereits bekannt sind, z. B. hergestellt werden nach den von E. Mosettig, Org. Reactions VIII, 218 ff. (1954) beschriebenen Methoden. Beispiele für geeignete Aldehyde der Formel IV sind: Benzaldehyd, 2-, 3- oder 4-Methyl-benzaldehyd, 2-, 3- oder 4-Ethyl-benzaldehyd, 2-, 3- oder 4-i-Propylbenzaldehyd, 2-, 3- oder 4- tert.-Butyl-benzaldehyd, 2-, 3- oder 4-Methoxy-benzaldehyd, 2-, 3- oder 4-i-Propoxy-benzaldehyd, 2-, 3- oder 4-Brom-benzaldehyd, 2-, 3- oder 4-Chlor-benzaldehyd, 2-, 3- oder 4-Fluor-benzaldehyd, 2-, 3- oder 4-Cyano-benzaldehyd, 2-, 3- oder 4-Trifluormethyl-benzaldehyd, 2-, 3- oder 4-Nitrobenzaldehyd, 2,4- oder 2,6-Dimethyl-benzaldehyd, 2,4- oder 2,6-Dichlor-benzaldehyd, 2,4- oder 2,6-Dibrom-benzaldehyd, 2,4- oder 2,6-Dinitro-benzaldehyd, 2,4- oder 2,6-Diethylbenzaldehyd, 3-Chlor-4-trifluormethyl-benzaldehyd, 3-Methyl-4-trifluormethyl-benzaldehyd, 3-Methoxy-4-chlor-benzaldehyd, 2-Methyl-4-cyano-benzaldehyd, Pyridin-2-aldehyd, Pyridin-3-aldehyd, Pyridin-4-aldehyd, 4-Methyl-pyridin-2-aldehyd, 5-Methyl-pyridin-2-aldehyd, 6-Methyl-pyridin-2-aldehyd, Thiophen-2-aldehyd, Thiophen-3-aldehyd, 5-Nitro-thiophen-2-aldehyd, 5-Methyl-thiophen-2-aldehyd, 5-Chlor-thiophen-2-aldehyd, 5-Methoxy-thiophen-2-aldehyd.

Die als Ausgangsverbindungen der Formel V benötigten Derivate des Acetessigesters können, soweit sie nicht bereits bekannt sind, nach den in Houben-Weyl, Methoden der Organischen Chemie, VII/4, (1968), 230 ff. und von H.O. House und S.K. Larson, J. Org. Chem., *33* (1968), 61, beschriebenen Verfahren hergestellt werden.

Geeignete Ausgangsverbindungen der Formel V sind z. B. Cyanaceton, Acetessigsäure-methylester, Acetessigsäure-ethyl-ester, Acetessigsäure-i-propyl-ester, Acetessigsäure-tert.-butyl-ester, Acetessigsäure-n-hexyl-ester, Acetessigsäureneo-pentyl-ester, Acetessigsäure-cyclohexyl-ester, Acetessigsäure-2-(dimethylamino)-ethyl-ester, Acetessigsäure-3-(diethylamino)-propyl-ester.

Die als Ausgangskomponenten benötigten Yliden-Verbindungen der Formeln VII und VIII können, soweit sie nicht bereits bekannt sind, hergestellt werden nach Org. Reactions XV, 204 ff. (1967).

Beispiele für geeignete Ausgangsverbindungen der Formeln VII und VIII sind: 2-Benzyliden-acetessigsäure-methyl-ester, 2-Benzyliden-acetessigsäure-cyclopentyl-ester, 2-(2-, 3- oder 4-Brombenzyliden)-acetessigsäure-ethyl-ester, 2-(2-, 3- oder 4-Nitro-benzyliden)acetessigsäure-i-propylester, 2-(2-, 3- oder 4-Trifluormethyl-benzyliden)-acetessigsäure-sec.-butyl-ester, 2-(2-, 3- oder 4-Ethyl-benzyliden)-acetessigsäure-neopentyl-ester, 2-(2-, 3- oder 4-tert.-Butyl-benzyliden)-acetessigsäure-(2-ethoxy-ethyl-ester, 2-(2-, 3- oder 4-Propoxy-benzyliden)- acetessigsäure-butyl-ester, 2-(2-, 3- oder 4-Chlor-benzyliden)-acetessigsäure-hexyl-ester, 2-(2-, 3- oder 4-Dichlor-benzyliden) acetessigsäure-methyl-ester, 2-(3-Methyl-4-Cyano-benzyliden)-acetessigsäure-i-propyl-ester.

Die Verbindungen der Formel II können, soweit sie nicht bereits bekannt sind, nach dem in Monatshefte für Chemie *113,* 781 ff. (1982) beschriebenen Verfahren hergestellt werden. Geeignete Ausgangsverbindungen der Formel II sind z. B. 5-Acetonyl-1,2,4-oxadiazol, 3-Methyl-5-acetonyl-1,2,4-oxadiazol, 3-Ethyl-5-acetonyl-1,2,4-oxadiazol, 3-tert.-Butyl-5-acetonyl-1,2,4-oxadiazol, 3-Methylthio-5-acetonyl-1,2,4-oxadiazol, 3-Benzyl-5-acetonyl-1,2,4-oxadiazol, 2-Acetonyl-1,3,4-oxadiazol, 5-Methyl-2-acetonyl-1,3,4-oxadiazol, 5-i-Propyl-2-acetonyl-1,3,4-oxadiazol, 3-Acetonyl-1,2,4-oxadiazol, 5-Ethyl-3-acetonyl-1,2,4-oxadiazol, 5-Ethylthio-3-acetonyl-1,2,4-oxadiazol, 5-Phenethyl-3-acetonyl-1,2,4-oxadiazol, 5-Acetonyl-1,2,4-thiadiazol, 3-Ethyl-5-acetonyl-1,2,4-thiadiazol, 3-Benzyl-5-acetonyl-1,2,4-thiadiazol.

Die 1,4-Dihydropyridine-Derivate der Formel I bilden, sofern sie basische Substituenten besitzen, mit anorganischen oder organischen Säuren Säureadditionssalze. Zur Bildung derartiger Säureadditionssalze sind anorganische und organische Säuren geeignet. Geeignete Säuren sind beispielsweise: Chlorwasserstoff, Bromwasserstoff, Naphthalindisulfonsäuren, insbesondere Naphthalin-1,5-disulfonsäure, Phosphor-, Salpeter-, Schwefel-, Oxal-, Milch-, Wein-, Essig-, Salicyl-, Benzoe-, Ameisen-, Propion-, Pivalin-, Diethylessig-, Malon-, Bernstein-, Pimelin-, Fumar-, Malein-, Apfel-, Sulfamin-, Phenylpropion-, Glucon-, Ascorbin-, Isonicotin-, Methansulfon-, p-Toluolsulfon-, Zitronen- oder Adipin-Säure. Pharmakologisch annehmbare Säureadditionssalze werden bevorzugt. Die Säureadditionssalze werden wie üblich durch Vereinigung der Komponenten, zweckmässigerweise in einem geeigneten Lösungs- oder Verdünnungsmittel, hergestellt. Bei der Synthese der Verbindungen der Formel I können zunächst die Säureadditionssalze im Zuge der Aufarbeitung anfallen. Aus den Säureadditionssalzen können die freien Verbindungen der allgemeinen Formel I gewünschenfalls in bekannter Weise, z. B. durch Auflösen oder Suspendieren in Wasser und Alkalischstellen, z. B. mit Natronlauge, und anschliessendes Isolieren, gewonnen werden.

Verbindungen der Formel I mit verschiedenen Bedeutungen von R und $R^2$ besitzen in der 4-Stellung des Dihydropyridinrings ein asymmetrisches Kohlenstoffatom. Diese Verbindungen treten daher als Racemat und in Form der optisch aktiven Enantiomeren auf. Für den Fall, dass die Verbindungen der Formel I mehr als ein asymmetrisches Atom besitzen, treten auch Diastereomere und ihre Mischungen auf. Mischungen von Diastereomeren und racemische Mischungen von Enantiomeren lassen sich nach bekannten Verfahren in die Einzelkomponenten auftrennen. Mischungen von Diastereomeren lassen sich z. B. durch fraktionierte Umkristallisation oder mit Hilfe von chromatographischen Verfahren in die Diastereomeren auftrennen. Ein Racemat lässt sich z. B. durch Umsetzung mit einer geeigneten enantiomeren Verbindung in ein diastereomeres Salzgemisch überführen, das dann z. B. durch fraktionierte Umkristallisation in die einzelnen diastereomeren Salze aufgetrennt wird. Die diastereomeren Salze werden dann in bekannter Weise in die enantiomeren Verbindungen aufgespalten.

Es ist bereits bekannt, dass bestimmte 1,4-Dihydropyridine interessante pharmakologische Eigenschaften aufweisen [F. Bossert, W. Vater Die Naturwissenschaften, *58*, 578 (1971)]. In der Regel stellen die bekannten wirksamen Verbindungen 1,4-Dihydropyridin-3,5-dicarbonester dar.

Überraschenderweise wurde nun gefunden, dass die neuen erfindungsgemässen Verbindungen der Formel I, die in 5-Position keine Esterfunktion aufweisen, besonders interessante kardiovaskuläre Wirkungen besitzen. Als hochwirksame Calciumantagonisten hemmen sie die durch Calcium in der Muskelzelle hervorgerufene Kontraktion und besitzen eine blutdrucksenkende und antianginöse Wirkung und können so z. B. zur Blutdrucksenkung und Herzentlastung beitragen. Die erfindungsgemässen Verbindungen lassen sich also unter anderem einsetzen bei Bluthochdruck und bei Angina pectoris und stellen somit eine Bereichung der Pharmazie dar.

Die Verbindungen der Formel I und ihre pharmakologisch annehmbaren Säureadditionssalze können daher am Menschen als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der Formel I oder eines Säureadditionssalzes davon neben üblichen pharmazeutisch einwandfreien Träger- und Zusatzstoffen enthalten. Die Zubereitungen enthalten normalerweise etwa 0,5 bis 90 Gewichtsprozent der therapeutisch wirksamen Verbindung.

Die Heilmittel können oral, z. B. in Form von Pillen, Tabletten, Lacktabletten, Dragees, Granulaten, Hart- und Weichgelatinekapseln, Lösungen, Sirupen, Emulsionen oder Suspensionen oder Aerosolmischungen verabreicht werden. Die Verabreichung kann aber auch rektal, z. B. in Form von Suppositorien, oder parenteral, z. B. in Form von Injektionslösungen, oder perkutan, z. B. in Form von Salben oder Tinkturen, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z. B. Lactose, Maisstärke oder Derivate davon, Talk, Stearinsäure oder deren Salze etc. verwenden. Trägerstoffe für Weichgelatinekapseln und Suppositorien sind z. B. Fette, Wachse, halbfeste und flüssige Polyole, natürliche oder gehärtete Öle etc. Als Trägerstoffe für die Herstellung von Lösungen und Sirupen eignen sich z. B. Wasser, Saccharose, Invertzucker, Glukose, Polyole etc. Als Trägerstoffe für die Herstellung von Injektionslösungen eignen sich z. B. Wasser, Alkohole, Glyzerin, Polyole, pflanzliche Öle etc.

Die pharmazeutischen Präparate können neben den Wirk- und Trägerstoffen noch Zusatzstoffe, wie z. B. Füllstoffe, Streck-, Spreng-, Binde-, Gleit-, Netz-, Stabilisierungs-, Emulgier-, Konservierungs-, Süss-, Färbe-, Geschmacks- oder Aromatisierungs-, Dickungs-, Verdünnungs-Mittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der Formel I und/oder ihrer pharmakologisch annehmbaren Säureadditionssalze und noch andere therapeutisch wirksame Stoffe enthalten.

Derartige andere therapeutisch wirksame Substanzen sind beispielsweise: β-Rezeptorenblokker, wie z. B. Propanolol, Pindolol, Metropolol; antianginöse Mittel, wie z. B. Carbochromen oder Molsidomin; Beruhigungsmittel, wie z. B. Barbitursäurederivate, 1,4-Benzodiazepine und Meprobamat; Diuretica, wie z. B. Chlorothiazid; das Herz tonisierende Mittel, wie z. B. Digitalispräparate; blutdrucksenkende Mittel, wie z. B. Hydralazin, Dihydralazin, Prazosin; Clonidin, Rauwolfia-Alkaloide; Mittel, die den Fettsäurespiegel im Blut senken, wie z. B. Bezafibrat, Fenofibrat; Mittel für die Thromboseprophylaxe, wie z. B. Phenprocoumon.

Die Verbindungen der Formel I, ihre pharmakologisch annehmbaren Säureadditionssalze und pharmazeutische Präparate, welche die Verbindungen der Formel I oder ihre pharmakologisch annehmbaren Säureadditionssalze als Wirkstoff enthalten, können am Menschen bei der Bekämpfung bzw. Vorbeugung von Erkrankungen verwendet werden, die durch ein Einströmen von Calcium in die Muskelzellen hervorgerufen werden und die durch die Verabreichung von Calciumantagonisten bekämpft werden können. So können sie beispielsweise als antihypertensive Heilmittel bei den verschiedenen Formen des Bluthochdrucks, bei der Bekämpfung bzw. Vorbeugung von Angina pectoris usw., sowie bei der Behandlung von cerebralen und peripheren Durchblutungsstörungen eingesetzt werden. Die Dosierung kann innerhalb weiter Grenzen variieren und ist in jedem einzelnen Fall den individuellen Gegebenheiten anzupassen. Im allgemeinen ist bei oraler Verabreichung eine Tagesdosis von etwa 0,01 bis 10 mg/kg, vorzugsweise 0,05 bis 5 mg/kg, Körpergewicht zur Erzielung wirksamer Ergebnisse angemessen. Bei intravenöser Applikation beträgt die Tagesdosis im allgemeinen etwa 0,001 bis 10 mg/kg, vorzugsweise 0,01 bis 5 mg/kg, Körpergewicht. Die Tagesdosis wird normalerweise, insbesondere bei der Applikation grösserer Mengen, in mehrere, z. B. 2, 3 oder 4, Teilverabreichungen aufgeteilt. Gegebenenfalls kann es je nach individuellem Verhalten erforderlich werden, von der angegebenen Tagesdosis nach oben oder unten abzuweichen.

*Beispiel 1:*

*1,4-Dihydro-2,6-dimethyl-4-(3-nitrophenyl)-5-(3-methyl-1,2,4-oxadiazol-5-yl)-pyridin-3-carbonsäuremethylester*

3,02 g 3-Nitrobenzaldehyd, 2,3 g Aminocrotonsäuremethylester und 2,8 g 3-Methyl-5-acetonyl-1,2,4-oxadiazol werden in 30 ml Isopropanl 5 Stunden zum Sieden erhitzt. Nach dem Erkaltenlassen wird der ausgefallene Feststoff abgesaugt und aus Ethanol umkristallisiert.

Fp = 239 bis 240° C

Ausbeute: 3,8 g

*Analyse:*

| | | | | |
|---|---|---|---|---|
| Berechnet: | C 58,4 | H 4,9 | N 15,1 | O 21,6% |
| Gefunden: | C 58,6 | H 4,9 | N 15,3 | O 21,1% |

In analoger Weise lassen sich herstellen:

a) 1,4-Dihydro-2,6-dimethyl-4-(2,3-dichlorphenyl)-5-(3-methyl-1,2,4-oxadiazol-5-yl)-pyridin-3-carbonsäuremethylester, Fp = 252 bis 254° C;

b) 1,4-Dihydro-2,6-dimethyl-4-(3-nitrophenyl)-5-(3-methyl-1,2,4-oxadiazol-5-yl)-pyridin-3-carbonsäure-(2-methoxy-ethyl)-ester, Fp = 214 bis 216° C;

c) 1,4-Dihydro-2,6-dimethyl-4-(2-nitrophenyl)-5-(3-methyl-1,2,4-oxadiazol-5-yl)-pyridin-3-carbonsäuremethylester, Fp = 220 bis 222° C;

d) 1,4-Dihydro-2,6-dimethyl-4-(2,3-dichlorphenyl)-5-(3-methyl-1,2,4-oxadiazol-5-yl)-pyridin-3-carbonsäure-(2-methoxy-ethyl)-ester, Fp = 196 bis 199° C;

e) 1,4-Dihydro-2,6-dimethyl-4-(pyrid-3-yl)-5-(3-methyl-1,2,4-oxadiazol-5-yl)-pyridin-3-carbonsäuremethylester, Fp = 248 bis 250° C;

f) 1,4-Dihydro-2,6-dimethyl-4-phenyl-5-(3-methyl-1,2,4-oxadiazol-5-yl)-pyridin-3-carbonsäuremethylester, Fp = 198 bis 200° C;

g) 1,4-Dihydro-2,6-dimethyl-4-(2-nitrophenyl)-5-(3-methyl-1,2,4-oxadiazol-5-yl)-pyridin-3-carbonsäure-(2-methoxy-ethyl)-ester, Fp = 177 bis 180° C;

h) 1,4-Dihydro-2,6-dimethyl-4-(3-methoxyphenyl)-5-(3-ethyl-1,2,4-oxadiazol-5-yl)-pyridin-3-carbonsäuremethylester, Fp = 180 bis 183° C;

i) 1,4-Dihydro-2,6-dimethyl-4-(3-nitrophenyl)-5-(3-tert.-butyl-1,2,4-oxadiazol-5-yl)-pyridin-3-carbonsäuremethylester, Fp = 197 bis 199° C;

k) 1,4-Dihydro-2,6-dimethyl-4-(2-chlorphenyl)-5-(3-methyl-1,2,4-oxadiazol-5-yl)-pyridin-3-carbonsäuremethylester, Fp = 211 bis 213° C;

l) 1,4-Dihydro-2,6-dimethyl-4-(3-chlorphenyl)-5-(3-methyl-1,2,4-oxadiazol-5-yl)-pyridin-3-carbonsäuremethylester, Fp = 228 bis 230° C;

m) 1,4-Dihydro-2,6-dimethyl-4-(4-chlorphenyl)-5-(3-methyl-1,2,4-oxadiazol-6-yl)-pyridin-3-carbonsäuremethylester, Fp = 227 bis 229° C;

n) 1,4-Dihydro-2,6-dimethyl-4-phenyl-5-(3-isopropyl-1,2,4-oxadiazol-5-yl)-pyridin-3-carbonsäure-(3-methoxy-propyl)-ester, Fp = 194 bis 196° C;

o) 1,4-Dihydro-2,6-dimethyl-4-(2,3-dichlorphenyl)-5-(tert.-butyl-1,2,4-oxadiazol-5-yl)-pyridin-3-carbonsäure-(2-butoxyethyl)-ester, Fp = 211 bis 213° C;

p) 1,4-Dihydro-2,6-dimethyl-4-(3-trifluormethyl-phenyl)-5-(3-benzyl-1,2,4-oxadiazol-5-yl)-pyridin-3-carbonsäure-(2-dimethylamino-ethyl)-ester, Fp = 205 bis 207° C;

q) 1,4-Dihydro-2,6-dimethyl-4-(2,3-dichlorphenyl)-5-(3-methyl-1,2,4-oxadiazol-5-yl)-pyridin-3-carbonsäure-(2-dimethylamino-ethyl)-ester, Fp = 170 bis 172° C;

r) 1,4-Dihydro-2,6-dimethyl-4-(2,3-dichlorphenyl)-5-(3-methyl-1,2,4-oxadiazol-5-yl)-pyridin-3-carbonsäure-(2-iso-propoxy-ethyl)-ester, Fp = 147 bis 149° C;

s) 1,4-Dihydro-2,6-dimethyl-4-(2,3-dichlorphenyl)-5-(3-methyl-1,2,4-oxadiazol-5-yl)-pyridin-3-carbonsäure-butyl-ester, Fp = 221 bis 223° C;

t) 1,4-Dihydro-2,6-dimethyl-4-(3-cyanophenyl)-5-(3-methyl-1,2,4-oxadiazol-5-yl)-pyridin-3-carbonsäure-(2-methoxy-ethyl)-ester, Fp = 175 bis 178° C;

u) 1,4-Dihydro-2,6-dimethyl-4-(2,3-dimethoxyphenyl)-5-(3-methyl-1,2,4-oxadiazol-5-yl)-pyridin-3-carbonsäure-(2-methoxy-ethyl)-ester, Fp = 165 bis 167° C;

v) 1,4-Dihydro-2,6-dimethyl-4-(2,3-dichlorphenyl)-5-(3-methyl-1,2,4-oxadiazol-5-yl)-pyridin-3-carbonsäure-isopropylester, Fp = 194 bis 196° C;

w) 1,4-Dihydro-2,6-dimethyl-4-(2,3-dichlorphenyl)-5-(3-methyl-1,2,4-oxadiazol-5-yl)-pyridin-3-carbonsäure-isobutylester, Fp = 158 bis 159° C;

x) 1,4-Dihydro-2,6-dimethyl-4-(3-chlorphenyl)-5-(3-methyl-1,2,4-oxadiazol-5-yl)-pyridin-3-carbonsäure-(2-methoxyethyl)-ester, Fp = 185 bis 187° C;
y) 1,4-Dihydro-2,6-dimethyl-4-(2,3-dimethoxyphenyl)-5-(1,3,4-oxadiazol-2-yl)-pyridin-3-carbonsäure-(2-methoxy-ethyl)-ester, Fp = 205 bis 207° C;
z) 1,4-Dihydro-2,6-dimethyl-4-(3-nitrophenyl)-5-(1,3,4-oxadiazol-2-yl)-pyridin-3-carbonsäure-isobutylester, Fp = 213 bis 215° C;
z1) 1,4-Dihydro-2,6-dimethyl-4-(3-nitrophenyl)-5-(2-aminocarbonyl-methylthio-1,3,4-oxadiazol-5-yl)-pyridin-3-carbonsäure-methylester, Fp = 246 bis 248° C;
z2) 1,4-Dihydro-2,6-dimethyl-4-(2,3-dichlorphenyl)-5-(2-aminocarbonylmethylthio-1,3,4-oxadiazol-5-yl)-pyridin-3-carbonsäuremethylester, Fp = 216 bis 218° C.

*Beispiel 2:*

*1,4-Dihydro-2,6-dimethyl-4-(3-nitrophenyl)-5-(1,3,4-oxadiazol-2-yl)-pyridin-3-carbonsäure-(2-methoxy-ethyl)-ester*

3,02 g 3-Nitrobenzaldehyd, 3,18 g Aminocrotonsäure-(2-methoxy-ethyl)-ester und 2,52 g 2-Acetonyl-1,3,4-oxadiazol werden in 30 ml Ethanol 5 Stunden zum Sieden erhitzt. Nach dem Erkaltenlassen wird der Feststoff abgesaugt und aus Essigester umkristallisiert. Nach nochmaligem Umkristallisieren aus Ethanol werden 2,3 g Feststoff erhalten. Fp = 208° C.

*Analyse:*

| | | | | |
|---|---|---|---|---|
| Berechnet: | C 57,0 | H 5,0 | N 14,0 | O 24,0% |
| Gefunden: | C 56,7 | H 5,2 | N 14,3 | O 23,8% |

In ähnlicher Weise können hergestellt werden:
a) 1,4-Dihydro-2,6-dimethyl-4-(3-nitrophenyl)-5-(1,3,4-oxadiazol-2-yl)-pyridin-3-carbonsäure-methylester, Fp = 266 bis 268° C;
b) 1,4-Dihydro-2,6-dimethyl-4-(2,3-dichlorphenyl)-5-(1,3,4-oxadiazol-2-yl)-pyridin-3-carbonsäure-methylester, Fp = 260 bis 262° C;
c) 1,4-Dihydro-2,6-dimethyl-4-(3-nitrophenyl)-5-(5-methyl-1,3,4-oxadiazol-2-yl)-pyridin-3-carbonsäure-methylester, Fp = 267 bis 269° C;
d) 1,4-Dihydro-2,6-dimethyl-4-(3-nitrophenyl)-5-(5-methyl-1,3,4-oxadiazol-2-yl)-pyridin-3-carbonsäure-(2-methoxy-ethyl)-ester, Fp = 234 bis 236° C;
e) 1,4-Dihydro-2,6-dimethyl-4-(2,3-dichlorphenyl)-5-(5-methyl-1,3,4-oxadiazol-2-yl)-pyridin-3-carbonsäure-methylester, Fp = 238 bis 240° C;
f) 1,4-Dihydro-2,6-dimethyl-4-(3-trifluormethylphenyl)-5-(5-methyl-1,3,4-oxadiazol-2-yl)-pyridin-3-carbonsäure-methylester, Fp = 207 bis 208° C;
g) 1,4-Dihydro-2,6-dimethyl-4-(2-chlorphenyl)-5-(5-methyl-1,2,4-oxadiazol-3-yl)-pyridin-3-carbonsäure-methylester, Fp = 214 bis 216° C;
h) 1,4-Dihydro-2,6-dimethyl-4-(3-methoxyphenyl)-5-(5-methyl-1,2,4-oxadiazol-3-yl)-pyridin-3-carbonsäure-isopropylester, Fp = 180 bis 182° C;
i) 1,4-Dihydro-2,6-dimethyl-4-(3-methylphenyl)-5-(5-methoxymethyl-1,2,4-oxadiazol-3-yl)-pyridin-3-carbonsäure-ethylester, Fp = 165 bis 168° C;
k) 1,4-Dihydro-2,6-dimethyl-4-(5-nitrothienyl)-5-(5-benzyl-1,2,4-oxadiazol-3-yl)-pyridin-3-carbonsäure-isobutylester, Fp = 201 bis 203° C;
l) 1,4-Dihydro-2,6-dimethyl-4-(2-nitrophenyl)-5-(3-methyl-1,2,4-thiadiazol-5-yl)-pyridin-3-carbonsäure-(2-methoxy-propyl)-ester, Fp = 185 bis 188° C;
m) 1,4-Dihydro-2,6-dimethyl-4-(3-cyanophenyl)-5-(3-methylthio-1,2,4-thiadiazol-5-yl)-pyridin-3-carbonsäure-n-butylester, Fp = 170 bis 172° C;
n) 1,4-Dihydro-2,6-dimethyl-4-(3-nitrophenyl)-5-(1,3,4-oxadiazol-2-yl)-pyridin-3-carbonsäure-isopropylester, Fp = 190 bis 192° C;
o) 1,4-Dihydro-2,6-dimethyl-4-(2-nitrophenyl)-5-(1,3,4-oxadiazol-2-yl)-pyridin-3-carbonsäure-(2-methoxy-ethyl)-ester, Fp = 168 bis 170° C;
p) 1,4-Dihydro-2,6-dimethyl-4-(2,3-dichlorphenyl)-5-(1,3,4-oxadiazol-2-yl)-pyridin-3-carbonsäure-(2-methoxy-ethyl)-ester, Fp = 173 bis 175° C;
q) 1,4-Dihydro-2,6-dimethyl-4-(3-nitrophenyl)-5-(1,3,4-oxadiazol-2-yl)-pyridin-3-carbonsäure-neopentylester, Fp = 196 bis 198° C;
r) 1,4-Dihydro-2,6-dimethyl-4-(3-nitrophenyl)-5-(3-benzyl-1,2,4-oxadiazol-5-yl)-pyridin-3-carbonsäure-methylester, Fp = 145 bis 147° C;
s) 1,4-Dihydro-2,6-dimethyl-4-(2,3-dichlorphenyl)-5-(3-benzyl-1,2,4-oxadiazol-5-yl)-pyridin-3-carbonsäure-methylester, Fp = 205 bis 207° C;
t) 1,4-Dihydro-2,6-dimethyl-4-(2-nitrophenyl)-5-(3-benzyl-1,2,4-oxadiazol-5-yl)-pyridin-3-carbonsäure-methylester, Fp = 189 bis 191° C;
u) 1,4-Dihydro-2,6-dimethyl-4-(3-trifluormethylphenyl)-5-(3-benzyl-1,2,4-oxadiazol-5-yl)-pyridin-3-carbonsäure-methylester, Fp = 179 bis 181° C;
v) 1,4-Dihydro-2,6-dimethyl-4-(3-nitrophenyl)-5-(3-benzyl-1,2,4-oxadiazol-5-yl)-pyridin-3-carbonsäure-(2-methoxy-ethyl)-ester, Fp = 108 bis 111° C;
w) 1,4-Dihydro-2,6-dimethyl-4-(2-chlorphenyl)-5-(3-benzyl-1,2,4-oxadiazol-5-yl)-pyridin-3-carbonsäure-methylester, Fp = 143 bis 145° C;
x) 1,4-Dihydro-2,6-dimethyl-4-(3-nitrophenyl)-5-(3-benzyl-1,2,4-oxadiazol-5-yl)-pyridin-3-carbonsäurenitril, Fp = 238 bis 240° C;
y) 1,4-Dihydro-2,6-dimethyl-4-(2,3-dichlorphenyl)-5-(3-benzyl-1,2,4-oxadiazol-5-yl)-pyridin-3-carbonsäure-(2-methoxy-ethyl)-ester, Fp = 101 bis 103° C;
z) 1,4-Dihydro-2,6-dimethyl-4-(pyrid-3-yl)-5-(3-benzyl-1,2,4-oxadiazol-5-yl)-pyridin-3-carbonsäure-isopropylester, Fp = 157 bis 160° C;
z1) 1,4-Dihydro-2,6-dimethyl-4-(2,3-dichlorphenyl)-5-(2-methyl-1,3,4-oxadiazol-5-yl)-pyridin-3-carbonsäure-(2-isopropoxy-ethyl)-ester, Fp = 163 bis 165° C;
z2) 1,4-Dihydro-2,6-dimethyl-4-(2,3-dichlorphenyl)-5-(1,3,4-oxadiazol-2-yl)-pyridin-3-carbonsäure-isopropylester, Fp = 166 bis 168° C.

*Beispiel 3:*

*1,4-Dihydro-2,6-dimethyl-3,5-di-(3-methyl-1,2,4-oxadiazol-5-yl)-4-(2,3-dichlorphenyl)-pyridin*

1,75 g 2,3-Dichlorbenzaldehyd, 2,8 g 3-Methyl-5-acetonyl-1,2,4-oxadiazol und 1,2 g 25%ige wässerige Ammoniaklösung werden in 50 ml Ethanol über Nacht zum Sieden erhitzt. Der Feststoff, der nach dem Abkühlen auf 0° C ausfällt, wird abgesaugt und aus Ethanol umkristallisiert. Fp = 255 bis 256° C.

*Analyse:*

Berechnet: C 54,6 H 4,1 N 16,7 O 7,7 Cl 17,0%

Gefunden: C 54,5 H 4,2 N 16,4 O 7,9 Cl 17,0%

In ähnlicher Weise lassen sich herstellen:

a) 1,4-Dihydro-2,6-dimethyl-3,5-di-(1,3,4-oxadiazol-2-yl)-4-(3-nitrophenyl)-pyridin, Fp = 254 bis 256° C;

b) 1,4-Dihydro-2,6-dimethyl-3,5-di-(5-methyl-1,2,4-oxadiazol-3-yl)-4-(p-tolyl)-pyridin, Fp = 304 bis 306° C;

c) 1,4-Dihydro-2,6-dimethyl-3,5-di-(3-methyl-1,2,4-thiadiazol-5-yl)-4-(2,3-dichlorphenyl)-pyridin, Fp = 231 bis 233° C;

d) 1,4-Dihydro-2,6-dimethyl-3,5-di-(5-methoxymethyl-1,2,4-oxadiazol-3-yl)-4-(2-trifluormethylphenyl)-pyridin, Fp = 196 bis 198° C;

e) 1,4-Dihydro-2,6-dimethyl-3,5-di-(3-benzyl-1,2,4-oxadiazol-5-yl)-4-(pyridin-2-yl)-pyridin, Fp = 248 bis 249° C;

f) 1,4-Dihydro-2,6-dimethyl-3,5-di-(4-methyl-5-ethoxy-carbonyl-1,3-thiazol-2-yl)-4-(3-nitrophenyl)-pyridin, Fp = 227 bis 229° C.

*Beispiel 4:*

*1,4-Dihydro-2,6-dimethyl-4-(3,5-dichlorphenyl)-5-(3-ethyl-1,2,4-oxadiazol-5-yl)-pyridin-3-carbonsäure-(2-methoxy-ethyl)-ester*

0,93 g 3-Ethyl-5-acetonyl-1,2,4-oxadiazol, 0,96 g β-Aminocrotonsäure-methoxyethylester und 0,95 g 3,5-Dichlorbenzaldehyd werden in 30 ml Isopropanol 8 Stunden am Rückfluss erhitzt. Nach dem Einengen wird der ölige Rückstand mit Diethylether verrührt, wobei allmählich Kristallisation eintritt. Der Feststoff wird abgesaugt und aus Isopropanol umkristallisiert. Ausbeute: 1,7 g; Fp = 178 bis 180° C.

*Analyse:*

Berechnet: C 55,9 H 4,9 N 9,3 O 14,2 Cl 15,7%

Gefunden: C 56,1 H 5,1 N 9,1 O 14,0 Cl 15,5%

In ähnlicher Weise lassen sich herstellen:

a) 1,4-Dihydro-2,6-dimethyl-4-(3-nitrophenyl)-5-(3-ethyl-1,2,4-oxadiazol-5-yl)-pyridin-3-carbonsäure-(2-methoxy-ethyl)-ester, Fp = 206 bis 208° C;

b) 1,4-Dihydro-2,6-dimethyl-4-(2,3-dichlorphenyl)-5-(3-ethyl-1,2,4-oxadiazol-5-yl)-pyridin-3-carbonsäure-(2-methoxy-ethyl)-ester, Fp = 153 bis 155° C;

c) 1,4-Dihydro-2,6-dimethyl-4-(2,3-dichlorphenyl)-5-(3-ethyl-1,2,4-oxadiazol-5-yl)-pyridin-3-carbonsäure-methylester, Fp = 190 bis 192° C;

d) 1,4-Dihydro-2,6-dimethyl-4-(4-nitrophenyl)-5-(3-ethyl-1,2,4-oxadiazol-5-yl)-pyridin-3-carbonsäure-(2-methoxy-ethyl)-ester, Fp = 171 bis 173° C;

e) 1,4-Dihydro-2,6-dimethyl-4-(2,4-dichlorphenyl)-5-(3-ethyl-1,2,4-oxadiazol-5-yl)-pyridin-3-carbonsäure-(2-methoxy-ethyl)-ester, Fp = 165 bis 167° C;

f) 1,4-Dihydro-2,6-dimethyl-4-(3,4-dichlorphenyl)-5-(3-ethyl-1,2,4-oxadiazol-5-yl)-pyridin-3-carbonsäure-(2-methoxy-ethyl)-ester, Fp = 178 bis 180° C;

g) 1,4-Dihydro-2,6-dimethyl-4-(3-bromphenyl)-5-(3-ethyl-1,2,4-oxadiazol-5-yl)-pyridin-3-carbonsäure-(2-methoxy-ethyl)-ester, Fp = 184 bis 186° C;

h) 1,4-Dihydro-2,6-dimethyl-4-(2,5-dimethylphenyl)-5-(3-ethyl-1,2,4-oxadiazol-5-yl)-pyridin-3-carbonsäure-(2-methoxy-ethyl)-ester, Fp = 157 bis 159° C;

i) 1,4-Dihydro-2,6-dimethyl-4-(2-chlor-6-nitrophenyl)-5-(3-ethyl-1,2,4-oxadiazol-5-yl)-pyridin-3-carbonsäure-(2-methoxy-ethyl)-ester, Fp = 162 bis 164° C;

k) 1,4-Dihydro-2,6-dimethyl-4-(3-nitrophenyl)-5-(2-ethyl-1,3,4-oxadiazol-5-yl)-pyridin-3-carbonsäure-(2-methoxy-ethyl)-ester, Fp = 224 bis 226° C;

l) 1,4-Dihydro-2,6-dimethyl-4-(3-nitrophenyl)-5-(2-ethyl-1,3,4-oxadiazol-5-yl)-pyridin-3-carbonsäure-methylester, Fp = 236 bis 238° C;

m) 1,4-Dihydro-2,6-dimethyl-4-(2,3-dichlorphenyl)-5-(2-ethyl-1,3,4-oxadiazol-5-yl)-pyridin-3-carbonsäure-(2-methoxy-ethyl)-ester, Fp = 166 bis 168° C;

n) 1,4-Dihydro-2,6-dimethyl-4-(3-nitrophenyl)-5-(2-éthyl-1,3,4-oxadiazol-5-yl)-pyridin-3-carbonsäure-isobutyl-ester, Fp = 204 bis 206° C;

o) 1,4-Dihydro-2,6-dimethyl-4-(3-nitrophenyl)-5-(1,3,4-oxadiazol-5-yl)-pyridin-3-carbonsäure(2-(N-benzyl-N-methylamino)-ethylester), Fp = 166 bis 168° C;

p) 1,4-Dihydro-2,6-dimethyl-4-(3-nitrophenyl)-5-(3-benzyl-1,2,4-oxadiazol-5-yl)-pyridin-3-carbonsäure-isobutylester, Fp = 121 bis 124° C;

q) 1,4-Dihydro-2,6-dimethyl-4-(3-nitrophenyl)-5-(3-benzyl-1,2,4-oxadiazol-5-yl)-pyridin-3-carbonsäure-tert.-butylester, Fp = 131 bis 133° C;

r) 1,4-Dihydro-2,6-dimethyl-4-(3-nitrophenyl)-5-(5-i-propylthio-1,3,4-oxadiazol-2-yl)-pyridin-3-carbonsäure-cyclohexylester, Fp = 189 bis 191° C;

s) 1,4-Dihydro-2,6-dimethyl-4-(3-nitrophenyl)-5-(5-cyclohexyl-1,2,4-oxadiazol-3-yl)-pyridin-3-carbonsäure-cyclopentylester, Fp = 201 bis 202° C;

*Beispiel 5:*

*1,4-Dihydro-2,6-dimethyl-4-(3-nitrophenyl)-5-(4-methyl-5-ethoxycarbonyl-thiazol-2-yl)-pyri-din-3-carbonsäuremethylester*

A) 2,3 g 2-(2-Aminopropen-1-yl)-4-methyl-5-ethoxycarbonylthiazol, 1,5 g 3-Nitrobenzaldehyd und 1,2 g Acetessigsäuremethylester werden in 30 ml Ethanol 3 Stunden am Rückfluss erhitzt. Die erkaltete Mischung wird mit Petrolether versetzt und über Nacht bei Raumtemperatur gerührt, wobei ein Niederschlag ausfällt; dieser wird aus Ethanol umkristallisiert. Ausbeute: 1,8 g; Fp = 203 bis 205° C.

*Analyse:*

Berechnet: C 59,6 H 5,2 O 21,7 N 6,3 S 7,2%

Gefunden: C 59,5 H 5,1 O 21,9 N 6,2 S 7,3%

B) Das als Ausgangsprodukt benötigte 2-(2-Aminopropen-1-yl)-4-methyl-5-ethoxycarbonyl-thiazol kann wie folgt hergestellt werden: 34,8 g Aminocrotonsäure-thioamid, 49,5 g 2-Chloracetessigsäureethylester und 45 ml Triethylamin werden 20 Minuten in 150 ml Ethanol am Rückfluss erhitzt. Dann lässt man die Mischung auf Raumtemperatur erkalten und verdünnt die Mischung mit Wasser, wobei ein Niederschlag ausfällt. Dieser wird abgesaugt und aus i-Propanol umkristallisiert. Ausbeute: 38,3 g; Fp = 98 bis 100° C.

*Analyse:*

Berechnet: C 53,1 H 6,2 N 12,4 O 14,2 S 14,2%

Gefunden: C 52,9 H 6,0 N 12,2 O 14,4 S 14,4%

In ähnlicher Weise lassen sich herstellen:

a) 1,4-Dihydro-2,6-dimethyl-4-(2,3-dichlorphenyl)-5-(4-methyl-5-ethoxycarbonyl-thiazol-2-yl)-pyridin-3-carbonsäure-isopropylester, Fp = 168 bis 170° C;

b) 1,4-Dihydro-2,6-dimethyl-4-(3-nitrophenyl)-5-(4-methyl-5-ethoxycarbonyl-thiazol-2-yl)-pyridin-3-carbonsäure-(2-methoxy-ethyl)-ester, Fp = 150 bis 152° C;

c) 1,4-Dihydro-2,6-dimethyl-4-(3-nitrophenyl)-5-(4-methyl-5-ethoxycarbonyl-thiazol-2-yl)-pyridin-3-carbonsäure-(2-(N-benzyl-N-methyl-amino)-ethylester), Fp = 148 bis 150° C;

d) 1,4-Dihydro-2,6-dimethyl-4-(3-nitrophenyl)-5-(4-phenyl-thiazol-2-yl)-pyridin-3-carbonsäure-methylester, Fp = 212 bis 215° C.

*Beispiel 6:*

*1,4-Dihydro-2,6-dimethyl-4-(2,3-dichlorphenyl)-5-(3-benzyl-1,2,4-oxadiazol-5-yl)-pyridin-3-carbonsäure-sec.-butylester*

1,75 g 2,3-Dichlorbenzaldehyd, 2,2 g 3-Acetonyl-1,2,4-oxadiazol, 1,6 g Acetessigsäure-sec.-butylester und 1,2 g 25%ige wässerige Ammoniaklösung werden in 50 ml Ethanol 6 Stunden zum Sieden erhitzt. Nach dem Einengen verbleibt ein öliger Rückstand, der mit Ether/Petrolether verrührt wird, und allmählich kristallisiert. Der erhaltene Feststoff wird dann aus Essigsäureethylester/Isopropanol umkristallisiert: Fp = 116 bis 117° C.

*Analyse:*

Berechnet: C 63,3 H 5,3 N 8,2 O 9,4 Cl 13,9%

Gefunden: C 63,1 H 5,4 N 8,1 O 9,6 Cl 13,7%

*Beispiel 7:*

*1,4-Dihydro-2,6-dimethyl-4-(2-nitrophenyl)-5-(4-methyl-5-ethoxycarbonyl-thiazol-2-yl)-pyridin-3-carbonsäure-methylester*

2,3 g 2-(2-Amino-propen-1-yl)-4-methyl-5-ethoxycarbonyl-thiazol und 2,5 g 2-Nitrobenzylidenacetessigsäure-methylester werden 4 Stunden lang in Isopropanol zum Sieden erhitzt. Beim Abkühlenlassen über Nacht fällt ein Niederschlag aus, der aus Ethanol umkristallisiert wird. Fp = 191 bis 192° C.

*Analyse:*

Berechnet: C 59,6 H 5,2 N 6,3 O 21,7 S 7,2%

Gefunden: C 59,4 H 5,3 N 6,2 O 21,9 S 7,1%

In den nachfolgenden beispielen werden pharmazeutische Präparate beschrieben:

*Beispiel 8:*

Gelatineweichkapseln, enthaltend 5 mg Wirkstoff pro Kapsel:

| | *pro Kapsel* |
|---|---|
| 1,4-Dihydro-2,6-dimethyl-4-(3-nitrophenyl)-5-(3-methyl-1,2,4-oxadiazol-5-yl)-pyridin-3-carbonsäuremethylester | 5 mg |
| Aus Kokosfett fraktioniertes Triglyceridgemisch | 150 mg |
| Kapselinhalt | 155 mg |

*Beispiel 9:*

Injektionslösung, enthaltend 1 mg Wirkstoff pro ml:

| | *pro ml* |
|---|---|
| 1,4-Dihydro-2,6-dimethyl-4-(3-nitrophenyl)-5-(3-methyl-1,2,4-oxadiazol-5-yl)-pyridin-3-carbonsäuremethylester | 1,0 mg |
| Polyethylenglycol 400 | 0,3 ml |
| Natriumchlorid | 2,7 mg |
| Wasser zu Injektionszwecken | ad 1,0 ml |

*Beispiel 10:*

Emulsion, enthaltend 10 mg Wirkstoff pro 5 ml:

| | *pro 100 ml* |
|---|---|
| 1,4-Dihydro-2,6-dimethyl-4-(3-nitrophenyl)-5-(1,3,4-oxadiazol-2-yl)-pyridin-3-carbonsäure-(2-methoxyethyl)ester | 0,2 g |
| Neutralöl | q.s. |

| | pro 100 ml |
|---|---|
| Natriumcarboxymethylcellulose | 0,6 g |
| Polyoxyethylen-stearat | q.s. |
| Glycerin rein | 0,2 bis 2,0 g |
| Geschmacksstoff | q.s. |
| Wasser (entsalzt oder destilliert) | ad 100 ml |

*Beispiel 11:*

Rektale Arzneiform, enthaltend 8 mg Wirkstoff pro Suppositorium

| | pro Suppositorium |
|---|---|
| 1,4-Dihydro-2,6-dimethyl-4-(3-nitrophenyl)-5-(1,3,4-oxadiazol-2-yl)-pyridin-3-carbonsäure-(2-methoxy-ethyl)ester | 8 mg |
| Suppositoriengrundmasse | ad 2 g |

*Beispiel 12:*

Tabletten, enthaltend 5 mg Wirkstoff pro Tablette

| | pro Tablette |
|---|---|
| 1,4-Dihydro-2,6-dimethyl-3,5-di(3-methyl-1,2,4-oxadiazol-5-yl)-4-(2,3-dichlorphenyl)-pyridin | 5 mg |
| Maisstärke (weiss) | 150 mg |
| Milchzucker | 60 mg |
| Mikrokristalline Cellulose | 50 mg |
| Polyvinylpyrrolidon | 20 mg |
| Magnesiumstearat | 2 mg |
| Natriumcarboxymethylstärke | 25 mg |
| | 312 mg |

*Beispiel 13:*

Dragees, enthaltend einen erfindungsgemässen Wirkstoff und einen anderen therapeutisch wirksamen Stoff

| | pro Dragee |
|---|---|
| 1,4-Dihydro-2,6-dimethyl-4-(3-nitrophenyl)-5-(3-methyl-1,2,4-oxadiazol-5-yl)pyridin-3-carbonsäuremethyl-ester | 6 mg |
| Propanolol | 40 mg |
| Milchzucker | 90 mg |
| Maisstärke | 90 mg |
| sec.-Calciumphosphat | 34 mg |
| Lösliche Stärke | 3 mg |
| Magnesiumstearat | 3 mg |
| Kolloidale Kieselsäure | 4 mg |
| | 270 mg |

*Beispiel 14:*

Dragees, enthaltend einen erfindungsgemässen Wirkstoff und einen anderen therapeutisch wirksamen Stoff

| | pro Dragee |
|---|---|
| 1,4-Dihydro-2,6-dimethyl-4-(3-nitrophenyl)-5-(3-methyl-1,2,4-oxadiazol-5-yl)pyridin-3-carbonsäuremethyl-ester | 6 mg |
| Molsidomin | 5 mg |
| Milchzucker | 90 mg |
| Maisstärke | 90 mg |
| sec.-Calciumphosphat | 34 mg |
| Lösliche Stärke | 3 mg |
| Magnesiumstearat | 3 mg |
| Kolloidale Kieselsäure | 4 mg |
| | 235 mg |

*Beispiel 15:*

Kapseln, enthaltend einen erfindungsgemässen Wirkstoff und einen anderen therapeutisch wirksamen Stoff

| | pro Kapsel |
|---|---|
| 1,4-Dihydro-2,6-dimethyl-4-(3-nitrophenyl)-5-(1,3,4-oxadiazol-2-yl)-pyridin-3-carbonsäure-(2-methoxy-ethyl)ester | 10 mg |
| Prazosin | 5 mg |
| Maisstärke | 185 mg |
| | 200 mg |

Die calciumantagonistische Wirkung der Verbindungen der Formel I wurde nach einer modifizierten Methode von Godfraind and Kaba [Arch. Int. Pharmacodyn. Ther., *196*, (Suppl) 35 bis 49, 1972] und von Schümann *et al.* (Naunyn-Schmiedeberg's Arch. Pharmacol., *289*, 409 bis 418, 1975) ermittelt. Dabei werden Spiralstreifen der Arteria pulmonalis des Meerschweinchens nach Äquilibrierung in calciumfreier Tyrodelösung mit 40 mmol Kalium depolarisiert. Ein Zusatz von 0,5 mmol $CaCl_2$ löst dann die Kontraktion aus. Die relaxierende Wirkung der Prüfsubstanz wird durch kumulative Zugabe in ½ log 10 abgestuften Konzentrationen ermittelt. Aus der Konzentrationswirkungskurve (Abszisse: -log mol/l Prüfsubstanz, Ordinate: % Hemmung der maximalen Kontraktion, Mittelwert von 4 bis 6 Gefässstreifen) wird die Konzentration der Prüfsubstanz ermittelt, welche die Kontraktion um 50% hemmt (= $IC_{50}$ mol/l). In der folgenden Tabelle sind die so erhaltenen $IC_{50}$-Werte angegeben. Wie der Vergleich mit dem $IC_{50}$-Wert $3 \cdot 10^{-9}$ für die bekannte Verbindung Nifedipin (= 1,4-Dihydro-2,6-dimethyl-4-(2-nitrophenyl)-pyridin-3,5-di-(carbonsäuremethylester)),vgl. DE-B-16 70 827, ergibt, liegen die Werte für die Verbindungen der Formel I z.T. erheblich günstiger.

*Tabelle*

| *Verbindung der Formel I gemäss Beispiel* | $IC_{50}$(mol/l) |
|---|---|
| 1t | $1{,}5 \cdot 10^{-9}$ |
| 1z | $1{,}5 \cdot 10^{-10}$ |
| 2b | $4 \cdot 10^{-10}$ |
| 2e | $1{,}6 \cdot 10^{-9}$ |
| 2n | $9 \cdot 10^{-11}$ |
| 2o | $3 \cdot 10^{-10}$ |
| 2r | $5 \cdot 10^{-10}$ |
| 2s | $7 \cdot 10^{-10}$ |
| 2t | $1 \cdot 10^{-9}$ |
| 2v | $7 \cdot 10^{-10}$ |
| 2w | $2{,}5 \cdot 10^{-9}$ |
| 2z1 | $1{,}5 \cdot 10^{-9}$ |
| 2z2 | $2 \cdot 10^{-10}$ |
| 3a | $2 \cdot 10^{-9}$ |
| 4b | $1{,}8 \cdot 10^{-9}$ |
| 4c | $2{,}4 \cdot 10^{-9}$ |
| 4k | $1 \cdot 10^{-9}$ |
| 4m | $1{,}5 \cdot 10^{-9}$ |
| 5b | $1{,}6 \cdot 10^{-9}$ |

**Patentansprüche** für die Vertragsstaaten BE, CH, DE, FR, GB, IT, LI, NL, SE

1. Neue substituierte 1,4-Dihydropyridine der Formel I

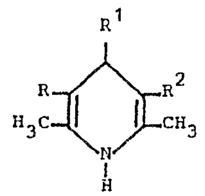

(I)

worin

R $-CO_2R^3$, Cyano oder eine der unter $R^2$ angegebenen Bedeutungen,

$R^1$ Pyridyl oder Thienyl, wobei der Pyridyl- oder Thienyl-Rest gegebenenfalls 1 oder 2 gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyano besitzt; Phenyl, das gegebenenfalls 1 oder 2 gleiche oder verschiedene Substituenten aus der Gruppe Halogen, Nitro, Cyano, Trifluormethyl, Alkyl mit 1 bis 4 C-Atomen, Alkoxy mit 1 bis 4 C-Atomen besitzt,

$R^2$ den Rest eines 5-gliedrigen Rings mit mindestens einer Doppelbindung und mindestens 2 Heteroatomen oder Heteroatomgruppierungen aus der Reihe O, N, NH, S, wobei der 5-gliedrige Ring gegebenenfalls 1 oder 2 gleiche oder verschiedene Substituenten aus der Gruppe Alkyl mit 1 bis 4 C-Atomen, Alkylthio mit 1 bis 4 C-Atomen, Aralkyl mit insgesamt 7 bis 9 C-Atomen, Alkoxyalkyl mit insgesamt 2 bis 5 C-Atomen, Cycloalkyl mit 5 oder 6 C-Atomen, Aminocarbonylmethylthio, Methoxy-carbonyl, Ethoxy-carbonyl, Phenyl besitzt,

$R^3$ Alkyl mit 1 bis 6 C-Atomen, Alkoxyalkyl mit 3 bis 8 C-Atomen, Dialkylaminoalkyl mit insgesamt 4 bis 9 C-Atomen, N-Aralkyl-N-alkylaminoalkyl mit insgesamt 10 bis 14 C-Atomen, Cycloalkyl mit 5 oder 6 C-Atomen

bedeuten, sowie ihre Säureadditionssalze.

2. Substituierte 1,4-Dihydropyridine nach Anspruch 1, dadurch gekennzeichnet, dass R = $-CO_2R^3$ und $R^3$ Alkyl mit 1 bis 5 C-Atomen, Alkoxyalkyl mit 1 bis 4 C-Atomen im Alkoxyteil und 2 bis 4 C-Atomen im Alkylteil, Dialkylaminoalkyl mit insgesamt 3 bis 6 C-Atomen, wobei jede der die Aminogruppe substituierenden Alkylgruppen 1 bis 3 C-Atome besitzt, bedeuten.

3. Substituierte 1,4-Dihydropyridine nach Anspruch 1 und/oder 2, dadurch gekennzeichnet, dass $R^1$ Phenyl bedeutet, das gegebenenfalls 1 oder 2 gleiche oder verschiedene Substituenten aus der Reihe Chlor, Brom, Fluor, Nitro, Cyano, Methyl, Methoxy, Trifluormethyl besitzt.

4. Substituierte 1,4-Dihydropyridine nach einem oder mehreren der Ansprüche 1 bis 3, dadurch gekennzeichnet, dass $R^2$ für Oxadiazolyl oder ein durch Methyl, Ethyl, i-Propyl, tert.-Butyl, Benzyl, Methylthio, i-Propylthio, Aminocarbonylmethylthio, Methoxymethyl, substituiertes Oxadiazolyl steht.

5. Substituierte 1,4-Dihydropyridine nach einem oder mehreren der Ansprüche 1 bis 4, dadurch gekennzeichnet, dass $R^2$ 1,3,4-Oxadiazol-2-yl, 5-Methyl-1,3,4-oxadiazol-2-yl, 5-Ethyl-1,3,4-oxadiazol-2-yl, 3-Methyl-1,2,4-oxadiazol-5-yl, 3-Ethyl-1,2,4-oxadiazol-5-yl, 3-Benzyl-1,2,4-oxadiazol-5-yl bedeutet.

6. Substituierte 1,4-Dihydropyridine nach einem oder mehreren der Ansprüche 1 bis 5, dadurch gekennzeichnet, dass $R^1$ 2-Nitrophenyl, 3-Nitrophenyl, 3-Cyanophenyl, 2-Chlorophenyl oder 2,3-Dichlorophenyl bedeutet.

7. Substituierte 1,4-Dihydropyridine nach einem oder mehreren der Ansprüche 1 bis 6, dadurch gekennzeichnet, dass R = $-CO_2R^3$ und $R^3$ Methyl, n-Propyl, i-Propyl, n-Butyl, i-Butyl, tert.-Butyl, 2-Methoxy-ethyl, 2-(i-Propoxy)-ethyl bedeuten.

8. 1,4-Dihydro-2,6-dimethyl-4-(3-nitrophenyl)-5-(1,3,4-oxadiazol-2-yl)-pyridin-3-carbonsäure-isobutylester.

9. 1,4-Dihydro-2,6-dimethyl-4-(2,3-dichlorphenyl)-5-(1,3,4-oxadiazol-2-yl)-pyridin-3-carbonsäure-methylester.

10. 1,4-Dihydro-2,6-dimethyl-4-(3-nitrophenyl)-5-(3-benzyl-1,2,4-oxadiazol-5-yl)-pyridin-3-carbonsäure-methylester.

11. 1,4-Dihydro-2,6-dimethyl-4-(2,3-dichlorphenyl)-5-(3-benzyl-1,2,4-oxadiazol-5-yl)-pyridin-3-carbonsäure-methylester.

12. 1,4-Dihydro-2,6-dimethyl-4-(3-nitrophenyl)-5-(3-benzyl-1,2,4-oxadiazol-5-yl)-pyridin-3-carbonsäure-(2-methoxy-ethyl)-ester.

13. 1,4-Dihydro-2,6-dimethyl-4-(3-nitrophenyl)-5-(1,3,4-oxadiazol-2-yl)-pyridin-3-carbonsäure-isopropylester.

14. 1,4-Dihydro-2,6-dimethyl-4-(2,3-dichlorphenyl)-5-(1,3,4-oxadiazol-2-yl)-pyridin-3-carbonsäure-isopropylester.

15. Analogieverfahren zur Herstellung der in einem oder mehreren der Ansprüche 1 bis 14 angegebenen Verbindungen der Formel I und ihrer Säureadditionssalze, dadurch gekennzeichnet, dass sie, ausgehend von Verbindungen der Formeln II bis IX

$H_3C-CO-CH_2-R^2$ (II), $H_3N$ (III), $R^1-CHO$ (IV),

$H_3C-CO-CH_2-R$ (V), $H_3C-C(NH_2)=CH-R$ (VI)

$R^1-CH=C(R^2)-CO-CH_3$ (VII), $R^1-CH=C(CO-CH_3)-R$ (VIII)

$H_3C-C(NH_2)=CH-R^2$ (IX)

dadurch hergestellt werden, dass

a) 2 mol einer Verbindung der Formel II und
 1 mol einer Verbindung der Formel IV und
 1 mol einer Verbindung der Formel III oder

b) 1 mol einer Verbindung der Formel II und
1 mol einer Verbindung der Formel III und
1 mol einer Verbindung der Formel IV und
1 mol einer Verbindung der Formel V oder
c) 1 mol einer Verbindung der Formel II und
1 mol einer Verbindung der Formel IV und
1 mol einer Verbindung der Formel VI oder
d) 1 mol einer Verbindung der Formel VI und
1 mol einer Verbindung der Formel VII oder
e) 1 mol einer Verbindung der Formel VIII und
1 mol einer Verbindung der Formel II und
1 mol einer Verbindung der Formel III oder
f) 1 mol einer Verbindung der Formel V und
1 mol einer Verbindung der Formel III und
1 mol einer Verbindung der Formel VII oder
g) 1 mol einer Verbindung der Formel VIII und
1 mol einer Verbindung der Formel IX oder
h) 2 mol einer Verbindung der Formel IX und
1 mol einer Verbindung der Formel IV oder
i) 1 mol einer Verbindung der Formel V und
1 mol einer Verbindung der Formel IV und
1 mol einer Verbindung der Formel IX oder
k) 1 mol einer Verbindung der Formel VII und
1 mol einer Verbindung der Formel IX

miteinander in einem Lösungsmittel bei Temperaturen von Raumtemperatur bis zur Rückflusstemperatur des Lösungsmittels umgesetzt und die erhaltene Verbindung der Formel I gewünschtenfalls in an sich bekannter Weise in ein Säureadditionssalz überführt wird.

16. Substituierte 1,4-Dihydropyridine der Ansprüche 1 bis 14 zur Verwendung als pharmakologische Wirkstoffe bei der Bekämpfung und Vorbeugung von Angina pectoris, Bluthochdruck, von cerebralen und peripheren Durchblutungsstörungen.

17. Verwendung der substituierten 1,4-Dihydropyridine der Ansprüche 1 bis 14 als Calciumantagonisten zur Herstellung pharmazeutischer Zubereitungen.

18. Pharmazeutisches Präparat, dadurch gekennzeichnet, dass es als Wirkstoff eine Verbindung der Ansprüche 1 bis 14 oder ein pharmazeutisch annehmbares Säureadditionssalz davon zusammen mit einem pharmazeutisch annehmbaren Trägerstoff und gegebenenfalls pharmazeutisch annehmbaren Zusatzstoffen und gegebenenfalls noch eine oder mehrere andere pharmakologische Wirkstoffe enthält.

**Patentansprüche** für den Vertragsstaat: AT

1. Analogieverfahren zur Herstellung neuer substituierter 1,4-Dihydropyridine der Formel I

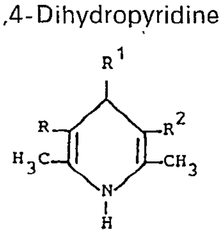

(I)

worin

R $-CO_2R^3$, Cyano oder eine der unter $R^2$ angegebenen Bedeutungen,

$R^1$ Pyridyl oder Thienyl, wobei der Pyridyl- oder Thienyl-Rest gegebenenfalls 1 oder 2 gleiche oder verschiedene Substituenten aus der Gruppe Alkyl mit 1 bis 4 C-Atomen, Alkoxy mit 1 bis 4 C-Atomen, Halogen, Trifluormethyl, Nitro, Cyano besitzt; Phenyl, das gegebenenfalls 1 oder 2 gleiche oder verschiedene Substituenten aus der Gruppe Halogen, Nitro, Cyano, Trifluormethyl, Alkyl mit 1 bis 4 C-Atomen, Alkoxy mit 1 bis 4 C-Atomen besitzt,

$R^2$ den Rest eines 5-gliedrigen Rings mit mindestens einer Doppelbindung und mindestens 2 Heteroatomen oder Heteroatomgruppierungen aus der Reihe O, N, NH, S, wobei der 5-gliedrige Ring gegebenenfalls 1 oder 2 gleiche oder verschiedene Substituenten aus der Gruppe Alkyl mit 1 bis 4 C-Atomen, Alkylthio mit 1 bis 4 C-Atomen, Aralkyl mit insgesamt 7 bis 9 C-Atomen, Alkoxyalkyl mit insgesamt 2 bis 5 C-Atomen, Cycloalkyl mit 5 oder 6 C-Atomen, Aminocarbonylmethylthio, Methoxy-carbonyl, Ethoxy-carbonyl, Phenyl besitzt,

$R^3$ Alkyl mit 1 bis 6 C-Atomen, Alkoxyalkyl mit 3 bis 8 C-Atomen, Dialkylaminoalkyl mit insgesamt 4 bis 9 C-Atomen, N-Aralkyl-N-alkylaminoalkyl mit insgesamt 10 bis 14 C-Atomen, Cycloalkyl mit 5 oder 6 C-Atomen bedeuten, sowie ihre Säureadditionssalze, dadurch gekennzeichnet, dass, ausgehend von Verbindungen der Formeln II bis VIII

$H_3C-CO-CH_2-R^2$, $H_3N$, $R^1-CHO$,
(II) (III) (IV)

$H_3C-CO-CH_2-R$, $H_3C-C(NH_2)=CH-R$,
(V) (VI)

$R^1-CH=C(R^2)-CO-CH_3$, $R^1-CH=C(CO-CH_3)-R$,
(VII) (VIII)

a) 2 mol einer Verbindung der Formel II und
1 mol einer Verbindung der Formel IV und
1 mol einer Verbindung der Formel III oder
b) 1 mol einer Verbindung der Formel II und
1 mol einer Verbindung der Formel III und
1 mol einer Verbindung der Formel IV und
1 mol einer Verbindung der Formel V oder
c) 1 mol einer Verbindung der Formel II und
1 mol einer Verbindung der Formel IV und
1 mol einer Verbindung der Formel VI oder
d) 1 mol einer Verbindung der Formel VII und
1 mol einer Verbindung der Formel VIII oder
e) 1 mol einer Verbindung der Formel V und
1 mol einer Verbindung der Formel IV und
1 mol einer Verbindung der Formel VIII oder

miteinander in einem Lösungsmittel bei Temperaturen von Raumtemperatur bis zur Rückflusstemperatur des Lösungsmittels umgesetzt und die erhaltene Verbindung der Formel I gewünschtenfalls in an sich bekannter Weise in ein Säureadditionssalz überführt wird.

2. Verfahren nach Anspruch 1, dadurch gekennzeichnet, dass die Ausgangsverbindungen so ausgewählt werden, dass Verbindungen der Formel I entstehen, bei denen R = $-CO_2R^3$ und $R^3$ Alkyl mit 1 bis 5 C-Atomen, Alkoxyalkyl mit 1 bis 4 C-Atomen im Alkoxyteil und 2 bis 4 C-Atomen im Alkylteil, Dialkylaminoalkyl mit insgesamt 3 bis 6 C-Atomen, wobei jede der die Aminogruppe substituierenden Alkylgruppen 1 bis 3 C-Atome besitzt, bedeuten.

3. Verfahren nach Anspruch 1 und/oder 2, dadurch gekennzeichnet, dass die Ausgangsverbindungen so ausgewählt werden, dass Verbindungen der Formel I entstehen, bei denen $R^1$ Phenyl bedeutet, das gegebenenfalls 1 oder 2 gleiche oder verschiedene Substituenten aus der Reihe Chlor, Brom, Fluor, Nitro, Cyano, Methyl, Methoxy, Trifluormethyl besitzt.

4. Verfahren nach einem oder mehreren der Ansprüche 1 bis 3, dadurch gekennzeichnet, dass die Ausgangsverbindungen so gewählt werden, dass Verbindungen der Formel I entstehen, bei denen $R^2$ für Oxadiazolyl oder ein durch Methyl, Ethyl, i-Propyl, tert.-Butyl, Benzyl, Methylthio, i-Propylthio, Aminocarbonylmethylthio, Methoxymethyl, substituiertes Oxadiazolyl steht.

5. Verfahren nach einem oder mehreren der Ansprüche 1 bis 4, dadurch gekennzeichnet, dass die Ausgangsverbindungen so ausgewählt werden, dass Verbindungen der Formel I entstehen, bei denen $R^2$ 1,3,4-Oxadiazol-2-yl, 5-Methyl-1,3,4-oxadiazol-2-yl, 5-Ethyl-1,3,4-oxadiazol-2-yl, 3-Methyl-1,2,4-oxadiazol-5-yl, 3-Ethyl-1,2,4-oxadiazol-5-yl, 3-Benzyl-1,2,4-oxadiazol-5-yl bedeutet.

6. Verfahren nach einem oder mehreren der Ansprüche 1 bis 5, dadurch gekennzeichnet, dass die Ausgangsverbindungen so ausgewählt werden, dass Verbindungen der Formel I entstehen, bei denen $R^1$ 2-Nitrophenyl, 3-Nitrophenyl, 3-Cyanophenyl, 2-Chlorophenyl oder 2,3-Dichlorophenyl bedeutet.

7. Verfahren nach einem oder mehreren der Ansprüche 1 bis 6, dadurch gekennzeichnet, dass die Ausgangsverbindungen so ausgewählt werden, dass Verbindungen der Formel I entstehen, bei denen R = $-CO_2R^3$ und $R^3$ Methyl, n-Propyl, i-Propyl, n-Butyl, i-Butyl, tert.-Butyl, 2-Methoxyethyl, 2-(i-Propoxy)-ethyl bedeuten.

8. Verfahren nach einem oder mehreren der Ansprüche 1 bis 7, dadurch gekennzeichnet, dass die Ausgangsverbindungen so ausgewählt werden, dass die Verbindung 1,4-Dihydro-2,6-dimethyl-4-(3-nitrophenyl)-5-(1,3,4-oxadiazol-2-yl)-pyridin-3-carbonsäure-isobutylester entsteht.

9. Verfahren nach einem oder mehreren der Ansprüche 1 bis 7, dadurch gekennzeichnet, dass die Ausgangsverbindungen so ausgewählt werden, dass die Verbindung 1,4-Dihydro-2,6-dimethyl-4-(2,3-dichlorphenyl)-5-(1,3,4-oxadiazol-2-yl)-pyridin-3-carbonsäure-methylester entsteht.

10. Verfahren nach einem oder mehreren der Ansprüche 1 bis 7, dadurch gekennzeichnet, dass die Ausgangsverbindungen so ausgewählt werden, dass die Verbindung 1,4-Dihydro-2,6-dimethyl-4-(3-nitrophenyl)-5-(3-benzyl-1,2,4-oxadiazol-5-yl)-pyridin-3-carbonsäure-methylester entsteht.

11. Verfahren nach einem oder mehreren der Ansprüche 1 bis 7, dadurch gekennzeichnet, dass die Ausgangsverbindungen so ausgewählt werden, dass die Verbindung 1,4-Dihydro-2,6-dimethyl-4-(2,3-dichlorphenyl)-5-(3-benzyl-1,2,4-oxa-diazol-5-yl)-pyridin-3-carbonsäure-methylester entsteht.

12. Verfahren nach einem oder mehreren der Ansprüche 1 bis 7, dadurch gekennzeichnet, dass die Ausgangsverbindungen so ausgewählt werden, dass die Verbindung 1,4-Dihydro-2,6-dimethyl-4-(3-nitrophenyl)-5-(3-benzyl-1,2,4-oxadiazol-5-yl)-pyridin-3-carbonsäure-(2-methoxyethyl)-ester entsteht.

13. Verfahren nach einem oder mehreren der Ansprüche 1 bis 7, dadurch gekennzeichnet, dass die Ausgangsverbindungen so ausgewählt werden, dass die Verbindung 1,4-Dihydro-2,6-dimethyl-4-(3-nitrophenyl)-5-(1,3,4-oxadiazol-2-yl)-pyridin-3-carbonsäure-isopropylester entsteht.

14. Verfahren nach einem oder mehreren der Ansprüche 1 bis 7, dadurch gekennzeichnet, dass die Ausgangsverbindungen so ausgewählt werden, dass die Verbindung 1,4-Dihydro-2,6-dimethyl-4-(2,3-dichlorphenyl)-5-(1,3,4-oxadiazol-2-yl)-pyridin-3-carbonsäure-isopropylester entsteht.

15. Verfahren nach einem oder mehreren der Ansprüche 1 bis 14, dadurch gekennzeichnet, dass 1 mol einer Verbindung der Formel II und 1 mol einer Verbindung der Formel IV und 1 mol einer Verbindung der Formel VI miteinander umgesetzt werden.

16. Verwendung der nach einem Verfahren der Ansprüche 1 bis 15 hergestellten substituierten 1,4-Dihydropyridine als pharmakologische Wirkstoffe zur Herstellung einer pharmazeutischen Zubereitung zur Bekämpfung und Vorbeugung von Angina pectoris, Bluthochdruck, von cerebralen und peripheren Durchblutungsstörungen.

17. Verwendung der nach einem Verfahren der Ansprüche 1 bis 15 hergestellten substituierten 1,4-Dihydropyridine als Calciumantagonisten zur Herstellung pharmazeutischer Zubereitungen.

**Claims** for the Contracting States BE, CH, DE, FR, GB, IT, LI, NL, SE

1. New substituted 1,4-dihydropyridines of the formula

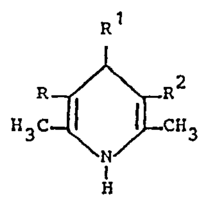

(I)

wherein

R denotes $-COR^3$, cyano, or has one of the meanings indicated under $R^2$,

$R^1$ denotes pyridyl or thienyl, the pyridyl or thienyl radical optionally possessing 1 or 2 identical or different substituents selected from the group comprising alkyl having 1 to 4 C atoms, alkoxy having 1 to 4 C atoms, halogen, trifluoromethyl, nitro or cyano; phenyl which optionally possesses 1 or 2 identical or different substituents selected from the group comprising halogen, nitro, cyano, trifluoromethyl, alkyl having 1 to 4 C atoms, alkoxy having 1 to 4 C atoms;

$R^2$ denotes the radical of a five-membered ring with at least one double bond and at least two heteroatoms or heteroatom groupings selected from the series comprising O, N, NH and S, the five-membered ring optionally possessing 1 or 2 identical or different substituents selected from the group comprising alkyl having 1 to 4 C atoms, alkylthio having 1 to 4 C atoms, aralkyl having a total of 7 to 9 C atoms, alkoxyalkyl having a total of 2 to 5 C atoms, cycloalkyl having 5 or 6 C atoms, aminocarbonyl-methylthio, methoxy-carbonyl, ethoxycarbonyl and phenyl;

$R^3$ denotes alkyl having 1 to 6 C atoms, alkoxyalkyl having 3 to 8 C atoms, dialkylaminoalkyl having a total of 4 to 9 C atoms, N-aralkyl-N-alkylaminoalkyl having a total of 10 to 14 C atoms, cycloalkyl having 5 or 6 C atoms, as well as their acid addition salts.

2. Substituted 1,4-dihydropyridines according to Claim 1, characterised in that R denotes $-CO_2R^3$ and $R^3$ denotes alkyl having 1 to 5 C atoms, alkoxyalkyl having 1 to 4 C atoms in the alkoxy portion and 2 to 4 C atoms in the alkyl portion, dialkylaminoalkyl having a total of 3 to 6 C atoms, each of the alkyl groups which substitute the amino group having 1 to 3 C atoms.

3. Substituted 1,4-dihydropyridines according to Claims 1 and/or 2, characterised in that $R^1$ denotes phenyl which optionally possesses 1 or 2 identical or different substituents selected from the group comprising chlorine, bromine, fluorine, nitro, cyano, methyl, methoxy and trifluoromethyl.

4. Substituted 1,4-dihydropyridines according to one or several of Claims 1 to 3, characterised in that $R^2$ represents oxadiazolyl or oxadiazolyl substituted by methyl, ethyl, i-propyl, tert.-butyl, benzyl, methylthio, i-propylthio, aminocarbonyl-methylthio or methoxymethyl.

5. Substituted 1,4-dihydropyridines according to one or several of Claims 1 to 4, characterised in that $R^2$ denotes 1,3,4-oxadiazol-2-yl, 5-methyl-1,3,4-oxadiazol-2-yl, 5-ethyl-1,3,4-oxadiazol-2-yl, 3-methyl-1,2,4-oxadiazol-5-yl, 3-ethyl-1,2,4-oxadiazol-5-yl, 3-benzyl-1,2,4-oxadiazol-5-yl.

6. Substituted 1,4-dihydropyridines according to one or several of Claims 1 to 5, characterised in that $R^1$ denotes 2-nitrophenyl, 3-nitrophenyl, 3-cyanophenyl, 2-chlorophenyl or 2,3-dichlorophenyl.

7. Substituted 1,4-dihydropyridines according to one or several of Claims 1 to 6, characterised in that R denotes $-CO_2R^3$ and $R^3$ denotes methyl, n-propyl, i-propyl, n-butyl, i-butyl, tert.-butyl, 2-methoxyethyl, 2-(i-propoxy)-ethyl.

8. 1,4-Dihydro-2,6-dimethyl-4-(3-nitrophenyl)-5-(1,3,4-oxadiazol-2-yl)-pyridine-3-carboxylic acid-isobutylester.

9. 1,4-Dihydro-2,6-dimethyl-4-(2,3-dichlorophenyl)-5-(1,3,4-oxadiazol-2-yl)-pyridine-3-carboxylic acid-methylester.

10. 1,4-Dihydro-2,6-dimethyl-4-(3-nitrophenyl)-5-(3-benzyl-1,2,4-oxadiazol-5-yl)-pyridine-3-carboxylic acid-methylester.

11. 1,4-Dihydro-2,6-dimethyl-4-(2,3-dichlorophenyl)-5-(3-benzyl-1,2,4-oxadiazol-5-yl)-pyridine-3-carboxylic acid-methylester.

12. 1,4-Dihydro-2,6-dimethyl-4-(3-nitrophenyl)-5-(3-benzyl-1,2,4-oxadiazol-5-yl)-pyridine-3-carboxylic acid-(2-methoxy-ethyl)-ester.

13. 1,4-Dihydro-2,6-dimethyl-4-(3-nitrophenyl)-5-(1,3,4-oxadiazol-2-yl)-pyridine-3-carboxylic acid-isopropylester.

14. 1,4-Dihydro-2,6-dimethyl-4-(2,3-dichlorophenyl)-5-(1,3,4-oxadiazol-2-yl)-pyridine-3-carboxylic acid-isopropylester.

15. Analogous process for the preparation of the compounds of the Formula I and their acid addition salts indicated in one or more of Claims 1 to 14, characterised in that, starting from compounds of the Formulae II to IX

$H_3C-CO-CH_2-R^2$, $H_3N$, $R^1-CHO$,
(II) (III) (IV)

$H_3C-CO-CH_2-R$,
(V)

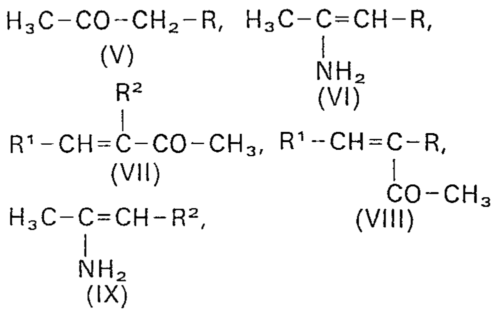

(VI) (VII) (VIII) (IX)

they are prepared in that

a) 2 mol of a compound of the formula II and 1 mol of a compound of the formula IV and 1 mol of a compound of the formula III, or
b) 1 mol of a compound of the formula II and 1 mol of a compound of the formula III and 1 mol of a compound of the formula IV and 1 mol of a compound of the formula V, or
c) 1 mol of a compound of the formula II and 1 mol of a compound of the formula IV and 1 mol of a compound of the formula VI, or
d) 1 mol of a compound of the formula VI and 1 mol of a compound of the formula VII, or
e) 1 mol of a compound of the formula VIII and 1 mol of a compound of the formula II and 1 mol of a compound of the formula III, or
f) 1 mol of a compound of the formula V and 1 mol of a compound of the formula III and 1 mol of a compound of the formula VII, or
g) 1 mol of a compound of the formula VIII and 1 mol of a compound of the formula IX, or
h) 2 mol of a compound of the formula IX and 1 mol of a compound of the formula IV, or
i) 1 mol of a compound of the formula V and 1 mol of a compound of the formula IV and 1 mol of a compound of the formula IX, or
k) 1 mol of a compound of the formula VII and 1 mol of a compound of the formula IX

are reacted together, in a solvent at temperatures from room temperature up to the reflux temperature of the solvent, and the resulting compound of the Formula I is converted, if desired, into an acid addition salt in a manner known per se.

16. Substituted 1,4-dihydropyridines of the Claims 1 to 14 for use as pharmacological active compounds for controlling and preventing angina pectoris, hypertension and disturbances of cerebral and peripheral blood flow.

17. Use of the substituted 1,4-dihydropyridines of the Claims 1 to 14 as calcium antagonists for preparing pharmaceutical preparations.

18. Pharmaceutical preparation characterised in that it contains, as active compound, a compound of Claims 1 to 14 or a pharmaceutically acceptable acid addition salt thereof, together with a pharmaceutically acceptable vehicle, and, where appropriate, pharmaceutically acceptable additives, and, where appropriate, one or more other pharmaceutical active compounds.

**Claims** for the Contracting State: AT

1. Analogous process for preparing new substituted 1,4-dihydropyridines of the formula

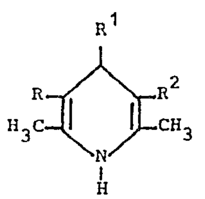

(I)

wherein

R denotes $-COR^3$, cyano, or has one of the meanings indicated under $R^2$,

$R^1$ denotes pyridyl or thienyl, the pyridyl or thienyl radical optionally possessing 1 or 2 identical or different substituents selected from the group comprising alkyl having 1 to 4 C atoms, alkoxy having 1 to 4 C atoms, halogen, trifluoromethyl, nitro or cyano; phenyl which optionally possesses 1 or 2 identical or different substituents selected from the group comprising halogen, nitro, cyano, trifluoromethyl, alkyl having 1 to 4 C atoms, alkoxy having 1 to 4 C atoms;

$R^2$ denotes the radical of a five-membered ring with at least one double bond and at least two heteroatoms or heteroatom groupings selected from the series comprising O, N, NH and S, the five-membered ring optionally possessing 1 or 2 identical or different substituents selected from the group comprising alkyl having 1 to 4 C atoms, alkylthio having 1 to 4 C atoms, aralkyl having a total of 7 to 9 C atoms, alkoxyalkyl having a total of 2 to 5 C atoms, cycloalkyl having 5 or 6 atoms, aminocarbonyl-methylthio, methoxy-carbonyl, ethoxycarbonyl and phenyl;

$R^3$ denotes alkyl having 1 to 6 C atoms, alkoxyalkyl having 3 to 8 C atoms, dialkylaminoalkyl having a total of 4 to 9 C atoms, N-aralkyl-N-alkylaminoalkyl having a total of 10 to 14 C atoms, cycloalkyl having 5 or 6 C atoms,

as well as their acid addition salts, characterised in that, starting from compounds of the Formulae II to VIII,

$H_3C-CO-CH_2-R^2$, $H_3N$, $R^1-CHO$,
(II) (III) (IV)

$H_3C-CO-CH_2-R$, $H_3C-C(NH_2)=CH-R$,
(V) (VI)

$R^1-CH=C(R^2)-CO-CH_3$, $R^1-CH=C(CO-CH_3)-R$,
(VII) (VIII)

they are prepared in that

a) 2 mol of a compound of the formula II and
1 mol of a compound of the formula IV and
1 mol of a compound of the formula III, or

b) 1 mol of a compound of the formula II and
1 mol of a compound of the formula III and
1 mol of a compound of the formula IV and
1 mol of a compound of the formula V, or

c) 1 mol of a compound of the formula II and
1 mol of a compound of the formula IV and
1 mol of a compound of the formula VI, or

d) 1 mol of a compound of the formula VII and
1 mol of a compound of the formula VIII, or

e) 1 mol of a compound of the formula V and
1 mol of a compound of the formula IV and
1 mol of a compound of the formula VIII,

are reacted together, in a solvent at temperatures from room temperature up to the reflux temperature of the solvent, and the resulting compound of the formula I is converted, if desired, into an acid addition salt in a manner known per se.

2. Process according to Claim 1, characterised in that the starting compounds are chosen such that compounds of the formula I are formed, wherein R denotes $-CO_2R^3$ and $R^3$ denotes alkyl having 1 to 5 C atoms, alkoxyalkyl having 1 to 4 C atoms in the alkoxy portion and 2 to 4 C atoms in the alkyl portion, dialkylaminoalkyl having a total of 3 to 6 C atoms, each of the alkyl groups which substitute the amino group having 1 to 3 C atoms.

3. Process according to Claims 1 and/or 2, characterised in that the starting compounds are chosen such that compounds of the formula I are formed, wherein $R^1$ denotes phenyl which optionally possesses 1 or 2 identical or different substituents selected from the group comprising chlorine, bromine, fluorine, nitro, cyano, methyl, methoxy and trifluoromethyl.

4. Process according to one or several of Claims 1 to 3, characterised in that the starting compounds are chosen such that compounds of the formula I are formed, wherein $R^2$ represents oxadiazolyl or oxadiazolyl substituted by methyl, ethyl, i-propyl, tert.-butyl, benzyl, methylthio, i-propylthio, aminocarbonylmethylthio or methoxymethyl.

5. Process according to one or several of Claims 1 to 4, characterised in that the starting compounds are chosen such that compounds of the

formula I are formed, wherein $R^2$ denotes 1,3,4-oxadiazol-2-yl, 5-methyl-1,3,4-oxadiazol-2-yl, 5-ethyl-1,3,4-oxadiazol-2-yl, 3-methyl-1,2,4-oxadiazol-5-yl, 3-ethyl-1,2,4-oxadiazol-5-yl, 3-benzyl-1,2,4-oxadiazol-5-yl.

6. Process according to one or several of Claims 1 to 5, characterised in that the starting compounds are chosen such that compounds of the formula I are formed, wherein $R^1$ denotes 2-nitrophenyl, 3-nitrophenyl, 3-cyanophenyl, 2-chlorophenyl or 2,3-dichlorophenyl.

7. Process according to one or several of Claims 1 to 6, characterised in that the starting compounds are chosen such that compounds of the formula I are formed, wherein R denotes $-CO_2R^3$ and $R^3$ denotes methyl, n-propyl, i-propyl, n-butyl, i-butyl, tert.-butyl, 2-methoxy-ethyl, 2-(i-propoxy)-ethyl.

8. Process according to one or several of Claims 1 to 7, characterised in the starting compounds are chosen such that the compound 1,4-dihydro-2,6-dimethyl-4-(3-nitrophenyl)-5-(1,3,4-oxadiazol-2-yl)-pyridin-3-carboxylic acid-isobutylester is formed.

9. Process according to one or several of Claims 1 to 7, characterised in the starting compounds are chosen such that the compound 1,4-dihydro-2,6-dimethyl-4-(2,3-dichlorophenyl)-5-(1,3,4-oxadiazol-2-yl)-pyridine-3-carboxylic acid-methylester is formed.

10. Process according to one or several of Claims 1 to 7, characterised in the starting compounds are chosen such that the compound 1,4-dihydro-2,6-dimethyl-4-1,4-dihydro-2,6-dimethyl-4-(3-nitrophenyl)-5-(3-benzyl-1,2,4-oxadiazol-5-yl)-pyridine-3-carboxylic acid-methylester is formed.

11. Process according to one or several of Claims 1 to 7, characterised in the starting compounds are chosen such that the compound 1,4-dihydro-2,6-dimethyl-4-(2-3-dichlorophenyl)-5-(3-benzyl-1,2,4-oxadiazol-5-yl)-pyridine-3-carboxylic acid-methylester is formed.

12. Process according to one or several of Claims 1 to 7, characterised in the starting compounds are chosen such that the compound 1,4-dihydro-2,6-dimethyl-4-(3-nitrophenyl)-5-(3-benzyl-1,2,4-oxadiazol-5-yl)-pyridine-3-carboxylic acid-(2-methoxy-ethyl)-ester is formed.

13. Process according to one or several of Claims 1 to 7, characterised in the starting compounds are chosen such that the compound 1,4-dihydro-2,6-dimethyl-4-(3-nitrophenyl)-5-(1,3,4-oxadiazol-2-yl)-pyridine-3-carboxylic acid-isopropylester is formed.

14. Process according to one or several of Claims 1 to 7, characterised in the starting compounds are chosen such that the compound 1,4-dihydro-2,6-dimethyl-4-(2,3-dichlorophenyl)-5-(1,3,4-oxadiazol-2-yl)-pyridine-3-carboxylic acid-isopropylester is formed.

15. Process according to one or several of Claims 1 to 14, characterised in that 1 mol of a compound of the formula II and 1 mol of a compound of the formula IV and 1 mol of a compound of the formula VI are reacted with one another.

16. Use of the substituted 1,4-dihydropyridines prepared according to a process of Claims 1 to 15 as pharmacological active compounds for controlling and preventing angina pectoris, hypertension and disturbances of cerebral and peripheral blood flow.

17. Use of the substituted 1,4-dihydropyridines prepared according to a process of the Claims 1 to 15 as calcium antagonists for preparing pharmaceutical preparations.

**Revendications** pour les Etats contractants BE, CH, DE, FR, GB, IT, LI, NL, SE

1. Nouvelles dihydro-1,4 pyridines substituées qui répondent à la formule I:

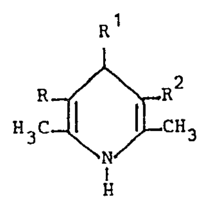

(I)

dans laquelle

R représente un radical $-CO_2R^3$ ou cyano ou a l'une des significations données ci-dessous pour $R_2$,

$R^1$ représente un radical pyridyle ou thiényle éventuellement porteur d'un substituant ou de deux substituants, identiques ou différents, pris dans l'ensemble constitué par les alkyles en $C_1$-$C_4$, les alcoxy en $C_1$-$C_4$, les halogènes et les radicaux trifluoro-méthyle, nitro et cyano, ou représente un radical phényle éventuellement porteur d'un substituant ou de deux substituants, identiques ou différents, pris dans l'ensemble constitué par les halogènes, les radicaux nitro, cyano et trifluorométhyle, les alkyles en $C_1$-$C_4$ et les alcoxy en $C_1$-$C_4$,

$R^2$ représente le radical d'un cycle pentagonal à au moins une double liaison et à au moins 2 hétéro-atomes ou groupement hétéro-atomiques pris dans la série O, N, NH et S, le cycle pentagonal portant éventuellement un substituant ou deux substituants, identiques ou différents, pris dans l'ensemble constitué par les alkyles en $C_1$-$C_4$, les alkylthio en $C_1$-$C_4$, les aralkyles renfermant au total de 7 à 9 atomes de carbone, les alcoxyalkyles renfermant au total de 2 à 5 atomes de carbone, les cyclo-alkyles en $C_5$ ou $C_6$ et les radicaux amino-carbonylméthylthio, méthoxycarbonyle, éthoxycarbonyle et phényle, et

$R^3$ représente un alkyle en $C_1$-$C_6$, alcoxyalkyle en $C_3$-$C_8$, un dialkylamino-alkyle contenant au total de 4 à 9 atomes de carbone, un N-aralkyl-N-alkyl-aminoalkyle contenant au total de 10 à 14 atomes de carbone, ou un cyclo-alkyle à 5 ou 6 atomes de carbone,

ainsi que les sels d'addition qu'elles forment avec des acides.

2. Dihydro-1,4 pyridines substituées selon la revendication 1, caractérisées en ce que R représente un radical $-CO_2R^3$ dans lequel $R^3$ désigne un radical alkyle contenant de 1 à 5 atomes de carbone, un radical alcoxyalkyle contenant de 1 à 4 atomes de carbone dans sa partie alcoxy et de 2 à 4 dans sa partie alkyle, ou un radical dialkylamino-alkyle contenant au total de 3 à 6 atomes de carbone, chacun des alkyles portés par le radical amino contenant de 1 à 3 atomes de carbone.

3. Dihydro-1,4 pyridines substituées selon l'une des revendications 1 et 2, caractérisées en ce que $R^1$ représente un radical phényle qui porte éventuellement un substituant ou deux substituants, identiques ou différents, pris dans l'ensemble constitué par le chlore, le brome, le fluor et les radicaux nitro, cyano, méthyle, méthoxy et trifluoro-méthyle.

4. Dihydro-1,4 pyridines substituées selon l'une des revendications 1 à 3, caractérisées en ce que $R^2$ représente un radical oxadiazolyle ou un radical oxadiazolyle qui porte un radical méthyle, éthyle, isopropyle, tert.-butyle, benzyle, méthylthio, isopropylthio, aminocarbonylméthylthio ou méthoxyméthyle.

5. Dihydro-1,4 pyridines substituées selon l'une des revendications 1 à 4, caractérisées en ce que $R^2$ représente un radical oxadiazole-1,3,4 yle-2, méthyl-5 oxadiazole-1,3,4 yle-2, éthyl-5 oxadiazole-1,3,4 yle-2, méthyl-3 oxadiazole-1,3,4 yle-5, éthyl-3 oxadiazole-1,2,4 yle-5 ou benzyl-3 oxadiazole-1,2,4 yle-5.

6. Dihydro-1,4 pyridines substituées selon l'une des revendications 1 à 5, caractérisées en ce que $R^1$ représente un radical nitro-2 phényle, nitro-3 phényle, cyano-3 phényle, chloro-2 phényle ou dichloro-2,3 phényle.

7. Dihydro-1,4 pyridines substituées selon l'une des revendications 1 à 6, caractérisées en ce que R représente un radical $-CO_2R^3$ dans lequel $R^3$ désigne un radical méthyle, n-propyle, isopropyle, n-butyle, isobutyle, tert.-butyle, méthoxy-2 éthyle ou isopropoxy-2 éthyle.

8. Ester isopropylique de l'acide dihydro-1,4 diméthyl-2,6 (nitro-3 phényl)-4 (oxadiazole-1,3,4 yle-2)-5 pyridine-carboxylique-3.

9. Ester méthylique de l'acide dihydro-1,4 diméthyl-2,6 (dichloro-2,3 phényl)-4 (oxadiazole-1,3,4 yle-2)-5 pyridine-carboxylique-3.

10. Ester méthylique de l'acide dihydro-1,4 diméthyl-2,6 (nitro-3 phényl)-4 (benzyl-3 oxadiazole-1,2,4 yle-5)-5 pyridine-carboxylique-3.

11. Ester méthylique de l'acide dihydro-1,4 diméthyl-2,6 (dichloro-2,3 phényl)-4 (benzyl-3 oxadiazole-1,2,4 yle-5)-5 pyridine-carboxylique-3.

12. Ester méthoxy-2 éthylique de l'acide dihydro-1,4 diméthyl-2,6 (nitro-3 phényl)-4 (benzyl-3 oxadiazole-1,2,4 yle-5)-5 pyridine-carboxylique-3.

13. Ester isopropylique de l'acide dihydro-1,4 diméthyl-2,6 (nitro-3 phényl)-4 (oxadiazole-1,3,4 yle-2)-5 pyridine-carboxylique-3.

14. Ester isopropylique de l'acide dihydro-1,4 diméthyl-2,6 (dichloro-2,3 phényl)-4 (oxadiazole-1,3,4 yle-2)-5 pyridine-carboxylique-3.

15. Procédé analogique pour la préparation des composés de formule I selon l'une des revendications 1 à 14, y compris de leurs sels d'addition d'acides, procédé caractérisé en ce que, partant de composés de formules II à IX:

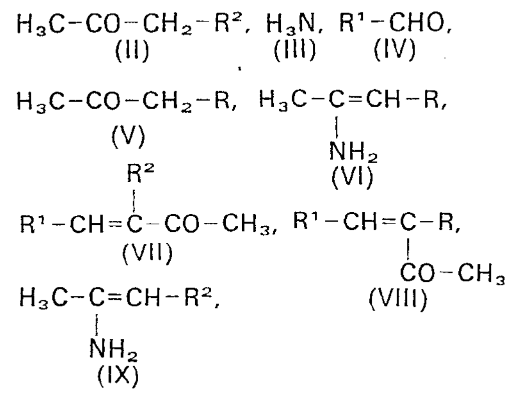

(II) (III) (IV) (V) (VI) (VII) (VIII) (IX)

on fait réagir ensemble:

a) 2 mol d'un composé de formule II, 1 mol d'un composé de formule IV, et 1 mol d'un composé de formule III, ou
b) 1 mol d'un composé de formule II, 1 mol d'un composé de formule III, 1 mol d'un composé de formule IV, et 1 mol d'un composé de formule V, ou
c) 1 mol d'un composé de formule II, 1 mol d'un composé de formule IV, et 1 mol d'un composé de formule VI, ou
d) 1 mol d'un composé de formule VI, et 1 mol d'un composé de formule VII, ou
e) 1 mol d'un composé de formule VIII, 1 mol d'un composé de formule II, et 1 mol d'un composé de formule III, ou
f) 1 mol d'un composé de formule V, 1 mol d'un composé de formule III, et 1 mol d'un composé de formule VIII, ou
g) 1 mol d'un composé de formule VIII, et 1 mol d'un composé de formule IX, ou
h) 2 mol d'un composé de formule IX, et 1 mol d'un composé de formule IV, ou
i) 1 mol d'un composé de formule V, 1 mol d'un composé de formule IV, et 1 mol d'un composé de formule IX, ou
k) 1 mol d'un composé de formule VII, et 1 mol d'un composé de formule IX,

dans un solvant, à des températures comprises entre la température ambiante et la température du reflux du solvant et, si on le désire, on transforme le composé de formule I obtenu, de manière connue, en un sel d'addition d'acide.

16. Dihydro-1,4 pyridines substituées selon l'une des revendications 1 à 14, destinées à être utilisées comme substances pharmacologiquement actives dans le traitement curatif et le traitement préventif de l'angine de poitrine, de l'hypertension et de troubles de l'irrigation sanguine cérébrale et périphérique.

17. Application des dihydro-1,4 pyridines substituées selon l'une des revendications 1 à 14 en

tant qu'antagonistes du calcium pour préparer des préparations pharmaceutiques.

18. Préparation pharmaceutique, caractérisée en ce qu'elle contient comme substance active un composé selon l'une des revendications 1 à 14, éventuellement sous la forme d'un sel d'addition d'acide acceptable du point de vue pharmaceutique, un excipient acceptable du point de vue pharmaceutique, éventuellement des additifs acceptables du point de vue pharmaceutique et, éventuellement, une ou plusieurs autres substances douées d'activités pharmacologiques.

**Revendications** pour l'Etat contractant: AT

1. Procédé analogique pour la préparation de nouvelle dihydro-1,4 pyridines substituées répondant à la formule I:

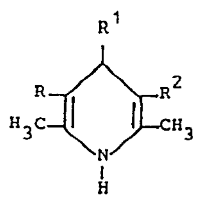

(I)

dans laquelle

R représente un radical $-CO_2R^3$ ou cyano ou a l'une des significations données ci-dessous pour $R_2$,

$R^1$ représente un radical pyridyle ou thiényle éventuellement porteur d'un substituant ou de deux substituants, identiques ou différents, pris dans l'ensemble constitué par les alkyles en $C_1$-$C_4$, les alcoxy en $C_1$-$C_4$, les halogènes et les radicaux trifluoro-méthyle, nitro et cyano, ou représente un radical phényle éventuellement porteur d'un substituant ou de deux substituants, identiques ou différents, pris dans l'ensemble constitué par les halogènes, les radicaux nitro, cyano et trifluorométhyle, les alkyles en $C_1$-$C_4$ et les alcoxy en $C_1$-$C_4$,

$R^2$ représente le radical d'un cycle pentagonal à au moins une double liaison et à au moins 2 hétéro-atomes ou groupements hétéro-atomiques pris dans la série O, N, NH et S, le cycle pentagonal portant éventuellement un substituant ou deux substituants, identiques ou différents, pris dans l'ensemble constitué par les alkyles en $C_1$-$C_4$, les alkylthio en $C_1$-$C_4$, les aralkyles renfermant au total de 7 à 9 atomes de carbone, les alcoxyalkyles renfermant au total de 2 à 5 atomes de carbone, les cyclo-alkyles en $C_5$ ou $C_6$ et les radicaux aminocarbonylméthylthio, méthoxycarbonyle, éthoxycarbonyle et phényle, et

$R^3$ représente un alkyle en $C_1$-$C_6$, un alcoxyalkyle en $C_3$-$C_8$, un dialkylamino-alkyle contenant au total de 4 à 9 atomes de carbone, un N-aralkyl-N-alkyl-aminoalkyle contenant au total de 10 à 14 atomes de carbone, ou un cyclo-alkyle à 5 ou 6 atomes de carbone,

ainsi que des sels d'addition qu'elles forment avec des acides, procédé caractérisé en ce que, partant de composés de formules II à VIII:

$H_3C-CO-CH_2-R^2$, (II) $H_3N$, (III) $R^1-CHO$, (IV)

$H_3C-CO-CH_2-R$, (V) $H_3C-C(NH_2)=CH-R$, (VI)

$R^1-CH=C(R^2)-CO-CH_3$, (VII) $R^1-CH=C(CO-CH_3)-R$, (VIII)

on fait réagir ensemble:

a) 2 mol d'un composé de formule II,
1 mol d'un composé de formule IV, et
1 mol d'un composé de formule III, ou

b) 1 mol d'un composé de formule II,
1 mol d'un composé de formule III,
1 mol d'un composé de formule IV, et
1 mol d'un composé de formule V, ou

c) 1 mol d'un composé de formule II,
1 mol d'un composé de formule IV, et
1 mol d'un composé de formule VI, ou

d) 1 mol d'un composé de formule VII, et
1 mol d'un composé de formule VIII, ou

e) 1 mol d'un composé de formule V,
1 mol d'un composé de formule IV, et
1 mol d'un composé de formule VIII,

dans un solvant, à une température comprise entre la température ambiante et la température du reflux du solvant et, si on le désire, on transforme le composé de formule I obtenu, de manière connue, en un sel d'addition d'acide.

2. Procédé selon la revendication 1, caractérisé en ce qu'on choisit les corps de départ de telle façon qu'il se forme des composés de formule I dans lesquels R représente un radical $-CO_2R^3$ dont le symbole $R^3$ désigne un alkyle contenant de 1 à 5 atomes de carbone, un alcoxy-alkyle contenant de 1 à 4 atomes de carbone dans sa partie alcoxy et de 2 à 4 dans sa partie alkyle, ou un dialkylamino contenant au total de 3 à 6 atomes de carbone, chacun des alkyles portés par le radical amino contenant de 1 à 3 atomes de carbone.

3. Procédé selon l'une des revendications 1 et 2, caractérisé en ce qu'on choisit les corps de départ de telle façon qu'il se forme des composés de formule I dans lesquels $R^1$ représente un radical phényle qui porte éventuellement un substituant ou deux substituants identiques ou différents, pris dans l'ensemble constitué par le chlore, le brome, le fluor et les radicaux nitro, cyano, méthyle, méthoxy et trifluoro-méthyle.

4. Procédé selon l'une des revendications 1 à 3, caractérisé en ce qu'on choisit les corps de départ de telle façon qu'il se forme des composés de formule I dans lesquels $R^2$ représente un radical oxadiazolyle ou un radical oxadiazolyle porteur d'un radical méthyle, éthyle, isopropyle, tert.-butyle, benzyle, méthylthio, isopropylthio, aminocarbonylméthylthio ou méthoxyméthyle.

5. Procédé selon l'une des revendications 1 à 4, caractérisé en ce qu'on choisit les corps de départ de telle façon qu'il se forme des composés de formule I dans lesquels $R^2$ représente un radical oxa-

diazole-1,3,4 yle-2, méthyle-5 oxadiazole-1,3,4 yle-2, éthyl-5 oxadiazole-1,3,4 yle-2, méthyl-3 oxadiazole-1,2,4 yle-5, éthyl-3 oxadiazole-1,2,4 yle-5 ou benzyl-3 oxadiazole-1,2,4 yle-5.

6. Procédé selon l'une des revendications 1 à 5, caractérisé en ce qu'on choisit les corps de départ de telle façon qu'il se forme des composés de formule I dans lesquels $R^1$ représente un radical nitro-2 phényle, nitro-3 phényle, cyano-3 phényle, chloro-2 phényle ou dichloro-2,3 phényle.

7. Procédé selon l'une des revendications 1 à 6, caractérisé en ce qu'on choisit les corps de départ de telle façon qu'il se forme des composés de formule I dans lesquels R représente un radical $-CO_2R^3$ dont le symbole $R^3$ désigne un radical méthyle, n-propyle, isopropyle, n-butyle, isobutyle, tert.-butyle, méthoxy-2 éthyle ou isopropoxy-2 éthyle.

8. Procédé selon l'une des revendications 1 à 7, caractérisé en ce qu'on choisit les corps de départ de telle façon qu'il se forme l'ester isobutylique de l'acide dihydro-1,4 diméthyl-2,6 (nitro-3 phényl)-4 (oxadiazole-1,3,4 yle-2)-5 pyridine-carboxylique-3.

9. Procédé selon l'une des revendications 1 à 7, caractérisé en ce qu'on choisit les corps de départ de telle façon qu'il se forme l'ester méthylique de l'acide dihydro-1,4 diméthyl-2,6 (dichloro-2,3 phényl)-4 (oxadiazole-1,3,4 yle-2)-5 pyridine-carboxylique-3.

10. Procédé selon l'une des revendications 1 à 7, caractérisé en ce qu'on choisit les corps de départ de telle façon qu'il se forme l'ester méthylique de l'acide dihydro-1,4 diméthyl-2,6 (nitro-3 phényl)-4 (benzyl-3 oxadiazole-1,2,4 yle-5)-5 pyridine-carboxylique-3.

11. Procédé selon l'une des revendications 1 à 7, caractérisé en ce qu'on choisit les corps de départ de telle façon qu'il se forme l'ester méthylique de l'acide dihydro-1,4 diméthyl-2,6 (dichloro-2,3 phényl)-4 (benzyl-3 oxadiazole-1,2,4 yle-5)-5 pyridine-carboxylique-3.

12. Procédé selon l'une des revendications 1 à 7, caractérisé en ce qu'on choisit les corps de départ de telle façon qu'il se forme l'ester méthoxy-2 éthylique de l'acide dihydro-1,4 diméthyl-2,6 (nitro-3 phényl)-4 (benzyl-3 oxadiazole-1,2,4 yle-5)-5 pyridine-carboxylique-3.

13. Procédé selon l'une des revendications 1 à 7, caractérisé en ce qu'on choisit les corps de départ de telle façon qu'il se forme l'ester isopropylique de l'acide dihydro-1,4 diméthyl-2,6 (nitro-3 phényl)-4 (oxadiazole-1,3,4 yle-2)-5 pyridine-carboxylique-3.

14. Procédé selon l'une des revendications 1 à 7, caractérisé en ce qu'on choisit les corps de départ de telle façon qu'il se forme l'ester isopropylique de l'acide dihydro-1,4 diméthyl-2,6 (dichloro-2,3 phényl)-4 (oxadiazole-1,3,4 yle-2)-5 pyridine-carboxylique-3.

15. Procédé selon l'une des revendications 1 à 14, caractérisé en ce qu'on fait réagir ensemble 1 mol d'un composé de formule II, 1 mol d'un composé de formule IV et 1 mol d'un composé de formule VI.

16. Application des dihydro-1,4 pyridines substituées qui ont été préparées par un procédé selon l'une des revendications 1 à 15, en tant que substances pharmacologiquement actives pour préparer une préparation pharmaceutique destinée au traitement curatif et au traitement préventif de l'angine de poitrine, de l'hypertension et de troubles de l'irrigation sanguine cérébrale et périphérique.

17. Application des dihydro 1-4 pyridines substituées qui ont été préparées par un procédé selon l'une des revendications 1 à 15, en tant qu'antagonistes du calcium pour préparer des préparations pharmaceutiques.